(19) Europäisches Patentamt
European Patent Office
Office européen des brevets

(11) EP 1 556 387 B1

(12) # FASCICULE DE BREVET EUROPEEN

(45) Date de publication et mention de la délivrance du brevet:
**04.04.2007 Bulletin 2007/14**

(21) Numéro de dépôt: **03809362.1**

(22) Date de dépôt: **17.10.2003**

(51) Int Cl.:
*C07D 498/04* (2006.01) *A61K 31/535* (2006.01)
*C07D 498/14* (2006.01) *C07D 265/38* (2006.01)

(86) Numéro de dépôt international:
**PCT/FR2003/003068**

(87) Numéro de publication internationale:
**WO 2004/037830 (06.05.2004 Gazette 2004/19)**

(54) **DERIVES DE BENZO (E) (1,4) OXAZINO (3,2-G) ISOINDOLE SUBSTI TUES, LEUR PROCEDE DE PREPARATION ET LES COMPOSITIONS PHARMACEUTIQUES QUI LES CONTIENNENT**

BENZO(E)(1,4)OXAZINO(3,2-G)ISOINDOL-DERIVATE, VERFAHREN ZU IHRER HERSTELLUNG UNDDIESE ENTHALTENDE PHARMAZEUTISCHE ZUSAMMENSETZUNGEN

SUBSTITUTED BENZOL E| 1,4| OXAZINO 3,2-G| ISOINDOLE DERIVATIVES, METHOD FOR PREPARING SAME AND PHARMACEUTICAL COMPOSITIONS CONTAINING SAME

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **18.10.2002 FR 0212964**

(43) Date de publication de la demande:
**27.07.2005 Bulletin 2005/30**

(73) Titulaire: **Les Laboratoires Servier**
**92415 Courbevoie Cedex (FR)**

(72) Inventeurs:
- **COUDERT, Gérard**
**F-45560 Saint Denis en Val (FR)**
- **LEPIFRE, Franck**
**F-45160 Olivet (FR)**
- **CAIGNARD, Daniel-Henri**
**F-78230 Le Pecq (FR)**
- **RENARD, Pierre**
**F-78150 Le Chesnay (FR)**
- **HICKMAN, John**
**F-75017 Paris (FR)**
- **PIERRE, Alain**
**F-78580 Les Alluets le Roi (FR)**
- **KRAUS-BERTHIER, Laurence**
**F-92700 Colombes (FR)**

(56) Documents cités:
**EP-A- 0 841 337**

- **NOBURU MOTOHASHI: "TEST FOR ANTITUMOR ACTIVITIES OF PHENOTHIAZINES AND PHENOXAZINES" PHARMACEUTICAL SOCIETY OF JAPAN. JOURNAL - YAKUGAKU ZASSHI, PHARMACEUTICAL SOCIETY OF JAPAN, TOKYO, JP, vol. 103, no. 3, 1983, pages 364-371, XP002917465 ISSN: 0031-6903**
- **MORGAN J ET AL: "COMPARISON OF PHOTODYNAMIC TARGETS IN A CARCINOMA CELL LINE AND ITS MITOCHONDRIAL DNA-DEFICIENT DERIVATIVE" PHOTOCHEMISTRY AND PHOTOBIOLOGY, OXFORD, GB, vol. 71, no. 6, 2000, pages 747-757, XP009008772 ISSN: 0031-8655**

Remarques:
Le dossier contient des informations techniques présentées postérieurement au dépôt de la demande et ne figurant pas dans le présent fascicule.

EP 1 556 387 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne les nouveaux dérivés benzo[e][1,4]oxazino[3,2-g]isoindole substitués, leur procédé de préparation et les compositions pharmaceutiques qui les contiennent. Les composés de la présente invention trouvent une utilisation thérapeutique intéressante grâce à leur activité antitumorale.
Dans la littérature, le J. Pharm. Sciences, 1974, 63(8), pp 1314-1316 décrit la synthèse de dérivés benzoxazinoquinoline qui possèdent des propriétés antitumorales. La demande de brevet EP 0841337 revendique des dérivés 7,12-dioxa-benzo[a]anthracénique substitués et décrit leurs propriétés anticancéreuses.
Les composés de la présente invention trouvent leur originalité à la fois dans leur structure et dans leur utilisation en tant qu'agent antitumoraux. Ils présentent également une biodisponibilité nettement supérieure par rapport à ceux de l'art antérieur.
Plus spécifiquement, la présente invention concerne les composés de formule (I) :

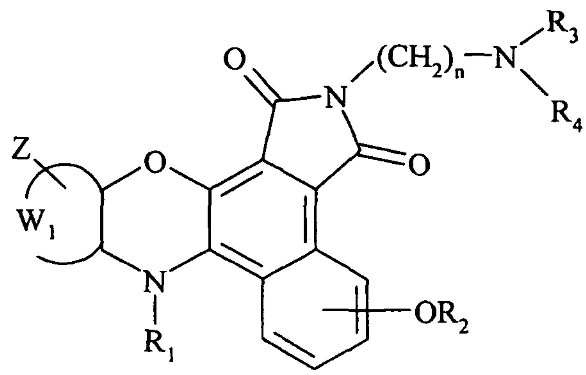

(I)

dans laquelle :

- **$W_1$** représente avec les atomes de carbone auxquels il est lié, un groupement phényle ou un groupement pyridinyle,
- **Z** représente un atome d'hydrogène,
- **$R_1$** représente un groupement choisi parmi -C(O)-$R_5$, atome d'hydrogène dans lesquels:

  ⇒ $R_5$ représente un groupement choisi parmi atome d'hydrogène, alkoxy ($C_1$-$C_6$) linéaire ou ramifié, ou aryloxy,

- **$R_2$** représente un atome d'hydrogène ou un groupement de formule -$CH_2CH_2O$-$R_8$ dans laquelle :

  $R_8$ représente un groupement choisi parmi atome d'hydrogène, -$S(O)_t$-$R_6$ (dans lequel $R_6$ représente un groupement alkyle ($C_1$-$C_6$) linéaire ou ramifiée, t représente 2),
  **$R_3$, $R_4$,** identiques ou différents, indépendamment l'un de l'autre, représentent un groupement alkyle ($C_1$-$C_6$) linéaire ou ramifié,

- n représente un entier compris entre 1 et 6 inclus

leurs énantiomères, diastéréoisomères, N-oxyde, ainsi que leurs sels d'addition à un acide ou à une base pharmaceutiquement acceptable,
étant entendu que par aryle, on comprend un groupement phényle, naphtyle, dihydronaphtyle, tétrahydronaphtyle, indényle ou indanyle, chacun de ces groupements étant éventuellement substitué par un ou plusieurs groupements, identiques ou différents, choisis parmi halogène, alkyle ($C_1$-$C_6$) linéaire ou ramifié, triahalogénoalkyle ($C_1$-$C_6$) linéaire ou ramifié, hydroxy, alkoxy ($C_1$-$C_6$) linéaire ou ramifié, et amino éventuellement substitué par un ou deux groupements alkyle ($C_1$-$C_6$) linéaire ou ramifié.
Parmi les acides pharmaceutiquement acceptables, on peut citer à titre non limitatif, les acides chlorhydrique,

bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer à titre non limitatif, l'hydroxyde de sodium, l'hydroxyde de potassium, la triéthylamine, etc...

Les composés préférés de l'invention sont les composés de formule (I) répondant plus particulièrement à la formule (IA) :

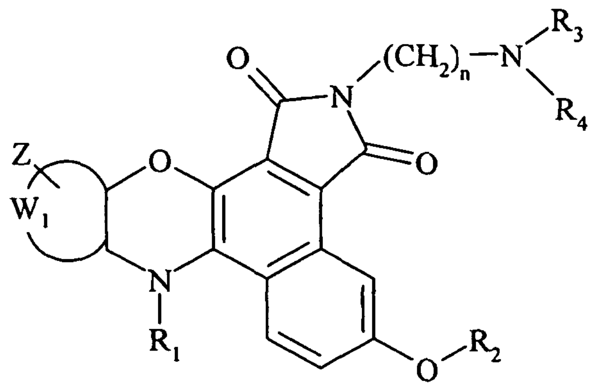

(IA)

dans laquelle $R_1$, $R_2$, $R_3$, $R_4$, $W_1$, Z et n sont tels que définis dans la formule (I).

Selon une deuxième variante avantageuse, les composés préférés de l'invention sont les composés de formule (I) répondant plus particulièrement à la formule (IB)

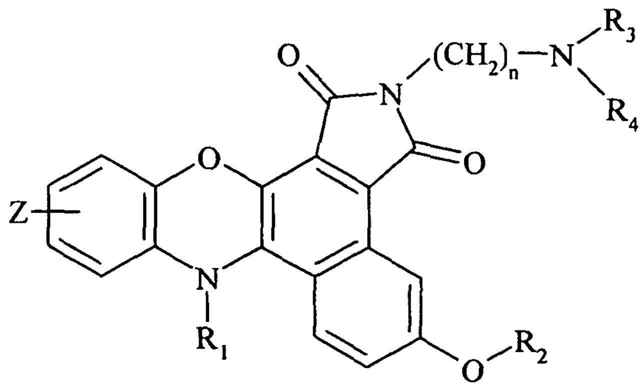

(IB)

dans laquelle $R_1$, $R_2$, $R_3$, $R_4$, Z et n sont tels que définis précédemment.

Selon une troisième variante avantageuse, les composés préférés de l'invention sont les composés de formule (I) répondant plus particulièrement à la formule (IC) :

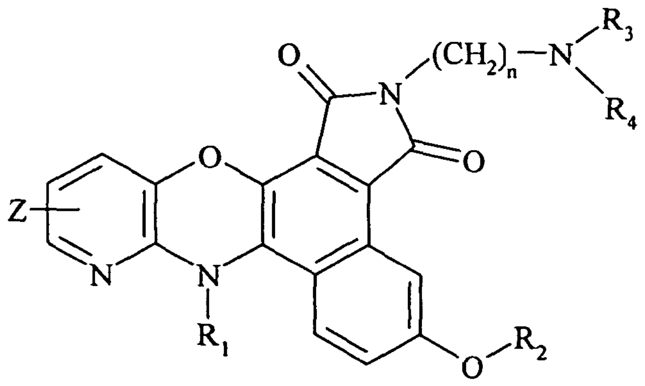

(IC)

dans laquelle $R_1$, $R_2$, $R_3$, $R_4$, Z et n sont tels que définis précédemment.

D'une façon avantageuse, le groupement $R_2$ préféré selon l'invention est l'atome d'hydrogène et le groupement $-CH_2CH_2O-R_8$ dans lequel $R_8$ représente plus particulièrement un atome d'hydrogène.

D'une façon très avantageuse, les composés préférés de l'invention sont ceux pour lesquels n représente le nombre entier 2.

Les composés préférés selon l'invention sont le :

- 2-[2-(diméthylamino)éthyl]-5-hydroxybenzo[a]pyrrolo[3,4-c]phénoxazine-1,3-dione,
- 2-[2-(diéthylamino)éthyl]-5-hydroxybenzo[a]pyrrolo[3,4-c]phénoxazine-1,3-dione,
- 2-[2-(diméthylamino)éthyl]-5-(2-hydroxyéthoxy)-2,3-dihydrobenzo[a]pyrrolo[3,4-c] phénoxazine-8-carbaldéhyde-1,3-dione,
- 2-[2-(diméthylamino)éthyl]-5-(2-hydroxyéthoxy)benzo[a]pyrrolo[3,4-c]phénoxazine-1,3-dione,
- 2-[2-(diméthylamino)éthyl]-5-(2-hydroxyéthylméthanesulfonate)benzo[a]pyrrolo [3,4-c]phénoxazine-1,3-dione,
- 2-[2-(diméthylamino)éthyl]-5-(2-hydroxyéthoxy)benzo[e]pyrido[2',3':5,6][1,4]-oxazino[3,2-g]isoindole-1,3-dione.

Les énantiomères, diastéréoisomères, N-oxydes, ainsi que leurs sels d'addition à un acide ou à une base pharmaceutiquement acceptable des composés préférés font partie intégrante de l'invention.

L'invention s'étend également au procédé de préparation des composés de formule (I), caractérisé en ce que l'on utilise comme produit de départ un composé de formule (II) :

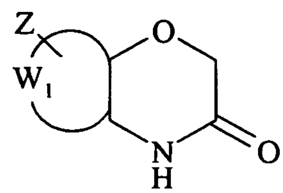

(II)

dans laquelle $W_1$ et Z sont tels que définis dans la formule (I),
composé de formule (II) dont on protège la fonction amine avec un groupement protecteur $P_G$ bien connu de l'homme de l'art pour conduire au composé de formule (III) :

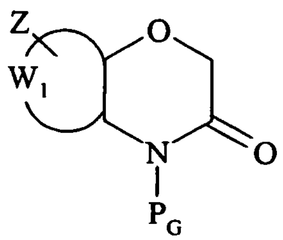

(III)

dans laquelle $P_G$ représente un groupement tert-butyloxycarbonyl ou phénoxycarbonyl et $W_1$ et Z sont tels que définis précédemment,
composé de formule (III) qui est traité par du diisopropylamidure de lithium suivi de chlorophosphate de diphényle pour conduire au composé de formule (IV) :

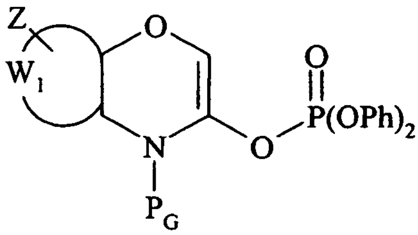

(IV)

dans laquelle $P_G$, $W_1$, et Z sont tels que définis précédemment,
composé de formule (IV) qui est traité, en présence de chlorure de bis(triphénylphosphine)palladium, par un composé de formule (V) :

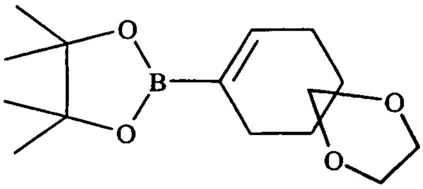

(V)

pour conduire au composé de formule (VI) :

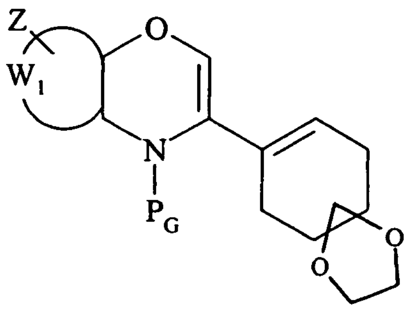

(VI)

dans laquelle $P_G$, $W_1$ et Z sont tels que définis précédemment,
composé de formule (VI) qui est :

- soit traité sous atmosphère inerte, par de l'acétylène dicarboxylate de diméthyle, pour conduire au composé de formule (VII) :

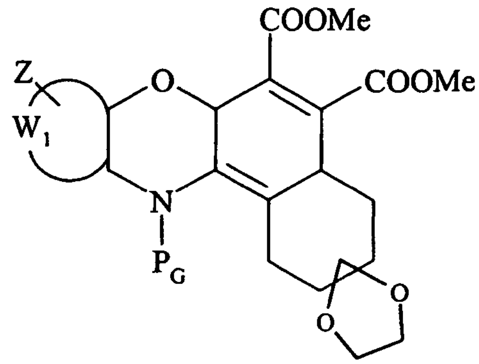

(VII)

dans laquelle $P_G$, $W_1$ et Z sont tels que définis précédemment,
composé de formule (VII) qui est :

- ♦ soit mis en présence de N-bromosuccinimide et de peroxyde de benzoyle, pour conduire au composé de formule (VIII) :

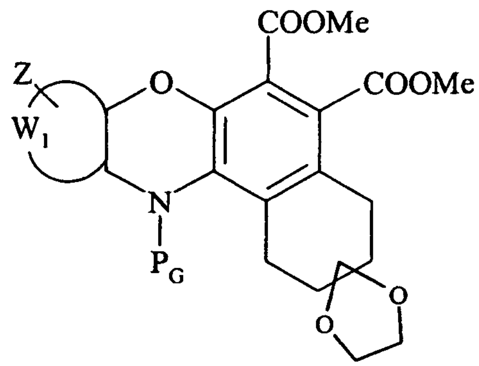

(VIII)

dans laquelle $P_G$, $W_1$ et Z sont tels que définis précédemment,
composé de formule (VIII) qui est soumis à l'action d'acide chlorhydrique pour conduire au composé de formule (IX) :

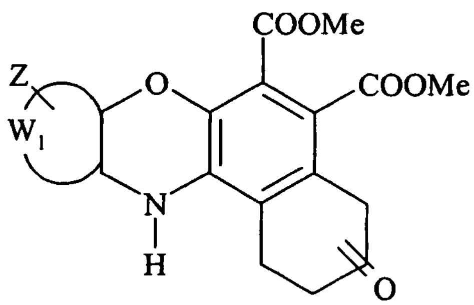

(IX)

dans laquelle $W_1$ et Z sont tels que définis précédemment,
composé de formule (IX) qui est soumis à l'action de di-tert-butyldicarbonate en présence de 4-diméthylaminopyridine, pour conduire au composé de formule (X) :

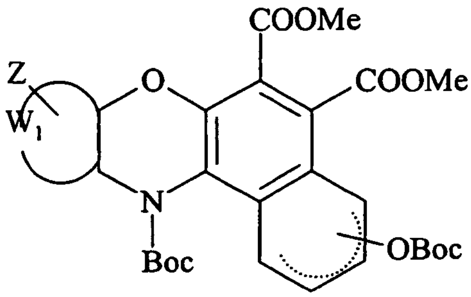

(X)

dans laquelle 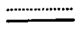 représente une liaison simple ou double, Boc représente un groupement tert-butyloxycarbonyl et $W_1$ et Z sont tels que définis précédemment,
composé de formule (X) qui est soumis à l'action de 2,3-dichloro-5,6-dicyano-1,4-benzoquinone, pour conduire au composé de formule (XI):

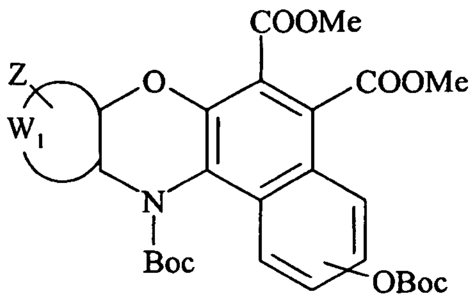

(XI)

dans laquelle Boc, $W_1$ et Z sont tels que définis précédemment,
composé de formule (XI) qui est soumis à l'action de méthanolate de sodium puis qui est hydrolysé pour conduire au composé de formule (XII) :

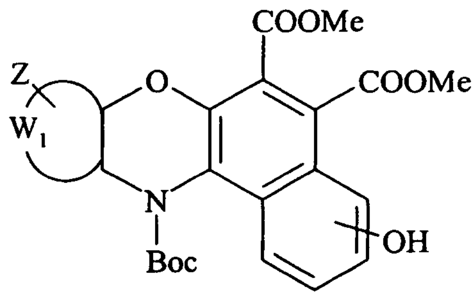

(XII)

dans laquelle Boc, $W_1$ et Z sont tels que définis précédemment,
composé de formule (XII) qui est soumis à l'action d'un composé de formule (XIII) :

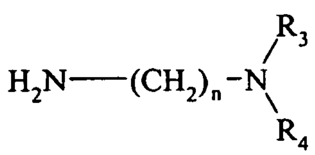

(XIII)

dans laquelle $R_3$, $R_4$ et n sont tels que définis dans la formule (I), pour conduire au composés de formule (I/a), cas particulier des composés de formule (I) :

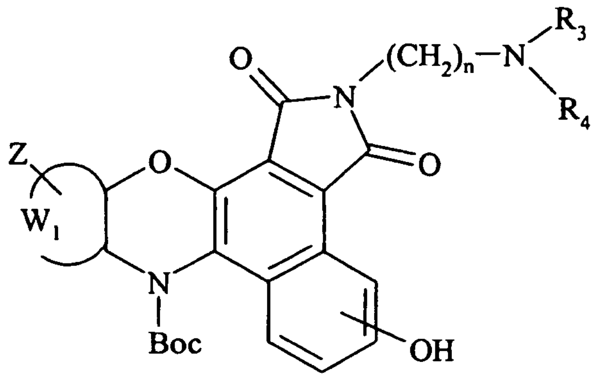

(I/a)

dans laquelle Boc, $R_3$, $R_4$, $W_1$, Z et n sont tels que définis précédemment,
composé de formule (I/a) qui est éventuellement soumis aux mêmes conditions de réaction que le composé de formule (VIII), pour conduire au composé de formule (I/b), cas particulier des composés de formule (I) :

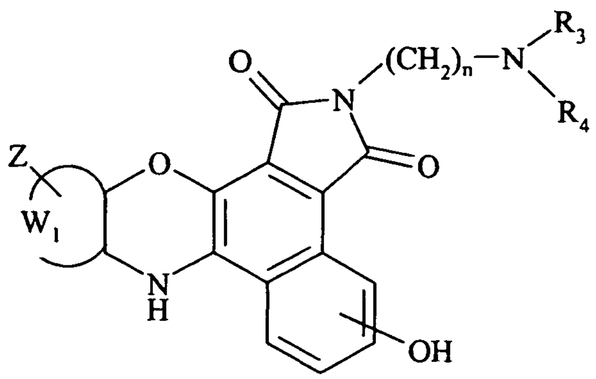

(I/b)

dans laquelle $R_3$, $R_4$, $W_1$, Z et n sont tels que définis précédemment,

♦ soit soumis aux mêmes conditions de réaction que le composé de formule (X), pour conduire au composé de formule (XIV) :

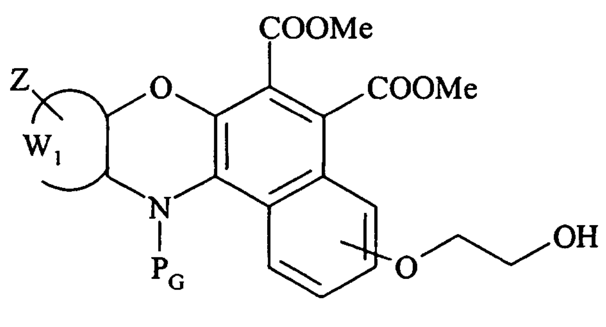

(XIV)

dans laquelle $P_G$, $W_1$, et Z sont tels que définis précédemment,
composé de formule (XIV) qui est soumis aux mêmes conditions de réaction que le composé de formule (XII), pour conduire au composé de formule (I/c), cas particulier des composés de formule (I) :

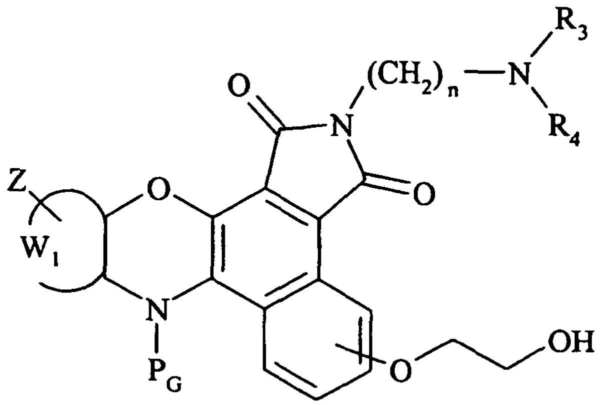

(I/c)

dans laquelle $P_G$, $R_3$, $R_4$, $W_1$, Z et n sont tels que définis précédemment, composés de formule (I/c) qui est :

■ soit éventuellement soumis à l'action d'acide formique pour conduire aux composés de formule (I/d) et (I/e), cas particuliers des composés de formule (I) :

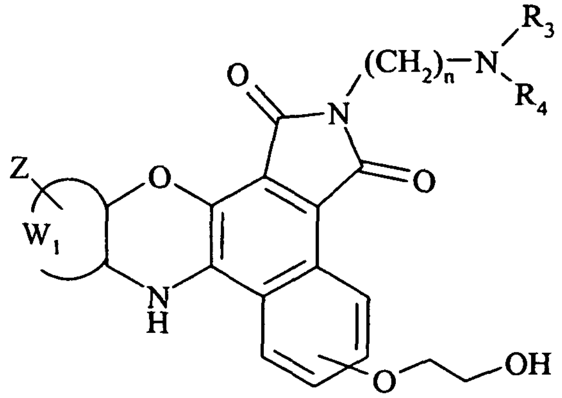

(I/d)

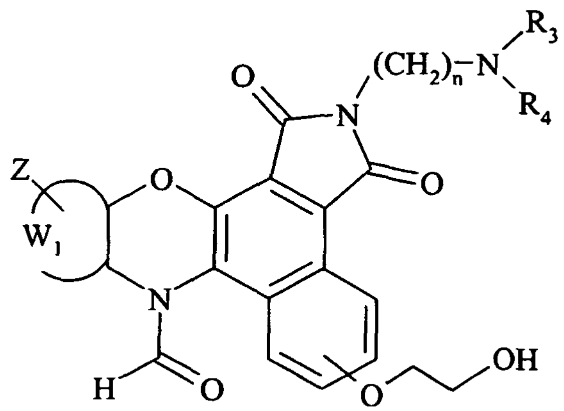

(I/e)

dans laquelle $R_3$, $R_4$, $W_1$, Z et n sont tels que définis précédemment,

■ soit éventuellement soumis à l'action d'un composé de formule (XV) :

$R_{8a}$-G (XV)

dans laquelle G représente un groupement partant et $R_{8a}$, différent de atome d'hydrogène, prend la même définition que $R_8$ dans la formule (I), pour conduire au composé de formule (I/f), cas particulier des composés de formule (I),

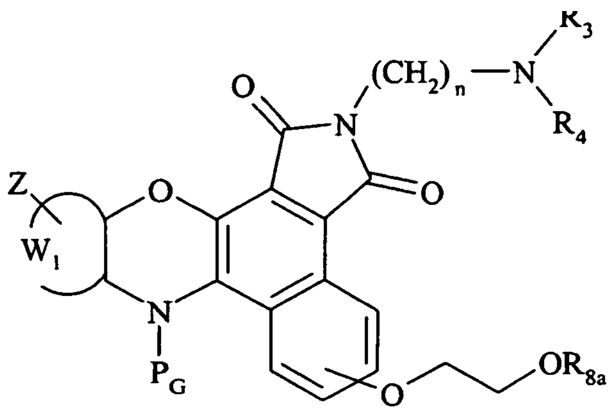

(I/f)

dans laquelle $P_G$, $R_3$, $R_4$, $R_{8a}$, $W_1$, Z et n sont tels que définis précédemment,
composés de formule (I/f) dont on déprotège éventuellement la fonction amine selon des méthodes classiques de la synthèse organique pour conduire au composé de formule (I/g), cas particulier des composés de formule (I) :

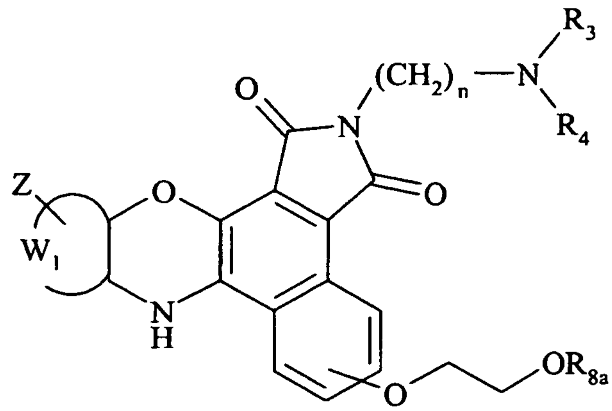

(I/g)

dans laquelle $R_3$, $R_4$, $R_{8a}$, $W_1$, Z et n sont tels que définis précédemment,
les composés de formule (I/b), (I/d) et (I/g) forment les composés de formule (I/h) :

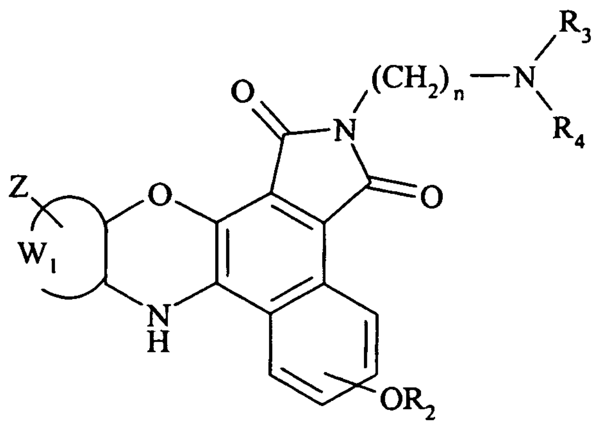

(I/h)

dans laquelle $R_2$, $R_3$, $R_4$, $W_1$, Z et n sont tels que définis précédemment,
composés de formule (I/h) qui sont éventuellement soumis à l'action d'un composé de formule (XVI) :

$R_{1a}$-G (XVI)

dans laquelle $R_{1a}$, différent de atome d'hydrogène, prend la même définition que $R_1$ dans la formule (I) et G est tel que défini précédemment, pour conduire au composé de formule (I/i), cas particulier des composés de formule (I) :

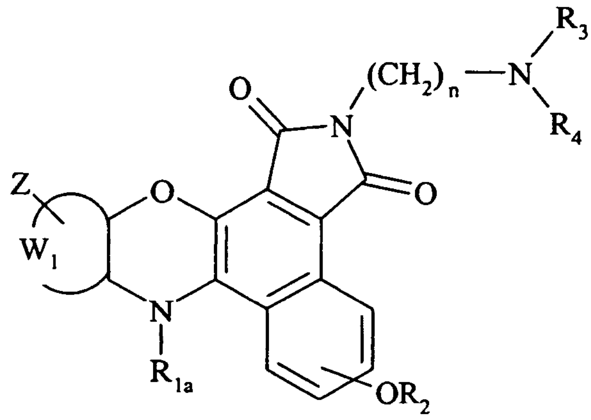

(I/i)

dans laquelle $R_{1a}$, $R_2$, $R_3$, $R_4$, $W_1$, Z et n sont tels que définis précédemment,

- soit traité avec du N-méthylmaléimide, pour conduire au composé de formule (XVII) :

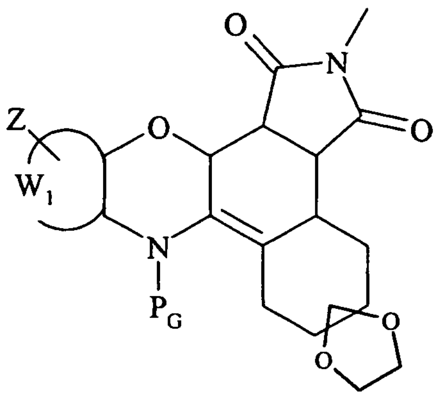

(XVII)

dans laquelle $P_G$, $W_1$ et Z sont tels que définis précédemment,
composé de formule (XVII) qui est soumis aux mêmes conditions de réaction que le composé de formule (VII) pour conduire au composé de formule (XVIII) :

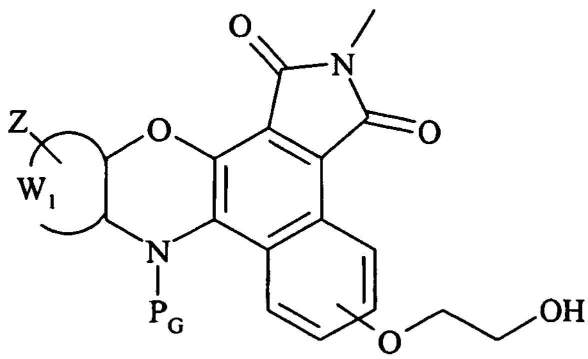

(XVIII)

dans laquelle $P_G$, $W_1$, et Z sont tels que définis précédemment, composé de formule (XVIII) qui est soumis aux

mêmes conditions de réactions que le composé de formule (XII) pour conduire au composé de formule (I/d) tel que décrit précédemment,

les composés de formule (I/a) à (I/i) forment l'ensemble des composés de formule (I), que l'on purifie le cas échéant, selon des techniques classiques de purification, qui peuvent si on le désire, être séparés en leurs différents isomères, selon une technique classique de séparation, et que l'on transforme, si on le souhaite, en leurs N-oxydes et, le cas échéant, en leurs sels d'addition à un acide ou à une base pharmaceutiquement acceptable.

L'invention concerne également les composés de formules (X), (XI) et (XIV) qui sont des intermédiaires de synthèse utiles dans la préparation des composés de formule (I).

Les composés de formule (II), (V), (XIII), (XV) et (XVI) sont soit des composés commerciaux, soit obtenus selon des méthodes classiques de la synthèse organique facilement accessible à l'homme du métier.

Les composés de formule (I) possèdent d'intéressantes propriétés pharmacologiques. Ils ont une excellente cytotoxicité *in vitro* non seulement sur des lignées leucémiques mais également sur des lignées de tumeurs solides, ils ont également une action sur le cycle cellulaire et sont actifs *in vivo,* sur un modèle leucémique. Ces propriétés permettent leur utilisation en thérapeutique en tant qu'agents antitumoraux.

Parmi les types de cancers que les composés de la présente invention peuvent traiter, on peut citer à titre non limitatif les adénocarcinomes et carcinomes, sarcomes, gliomes et leucémies.

La présente invention a également pour objet les compositions pharmaceutiques contenant les produits de formule (I), ses énantiomères, diastéréoisomères, N-oxydes ou un de leurs sels d'addition à une base ou un acide pharmaceutiquement acceptables, seul ou en combinaison avec un ou plusieurs excipients ou véhicules inertes, non toxiques.

Parmi les compositions pharmaceutiques selon l'invention, il sera cité plus particulièrement celles qui conviennent pour l'administration orale, parentérale, nasale, rectale, perlinguale, oculaire ou respiratoire, et notamment les comprimés simples ou dragéifiés, les comprimés sublinguaux, les sachets, les paquets, les gélules, les glossettes, les tablettes, les suppositoires, les crèmes, les pommades, les gels dermiques, les préparations injectables ou buvables, les aérosols, les gouttes oculaires ou nasales.

De part les propriétés pharmacologiques caractéristiques des composés de formule (I), les compositions pharmaceutiques contenant comme principe actif lesdits composés de formule (I), sont donc particulièrement utiles pour le traitement des cancers.

La posologie utile varie selon l'âge et le poids du patient, la voie d'administration, la nature de l'indication thérapeutique et des traitements éventuellement associés et s'échelonne entre 0,1 et 400 mg par jour, en une ou plusieurs administrations.

Les exemples suivants illustrent l'invention et ne la limitent en aucune façon. Les produits de départ utilisés sont des produits connus ou préparés selon des modes opératoires connus.

Les structures des composés décrits dans les exemples ont été déterminées selon les techniques spectrophotométriques usuelles (infrarouge, résonance magnétique nucléaire, spectrométrie de masse, ...).

**PREPARATION A : 3-[(diphénoxyphosphoryl)oxy]-4*H*-1,4-benzoxazine-4-carboxylate de tert-butyle**

*Stade A : 2,3-dihydro-4H-1,4-benzoxazine-3-one-4-carboxylate de tert-butyle*

Sous atmosphère inerte, 73 mmol de 2*H*-1,4,benzoxazine-3-one sont dissous dans 100 ml d'acétonitrile en présence de 3,65 mmol de 4-diméthylaminopyridine et 80 mmol de dicarbonate de di-tert-butyle. Le milieu est laissé sous agitation pendant 4 heures. Après concentration, le résidu est repris par l'acétate d'éthyle. La phase organique est lavée par une solution saturée de chlorure de sodium, séchée sur sulfate de magnésium, filtrée et concentrée. Après évaporation du solvant et purification par chromatographie sur gel de silice (éther de pétrole/acétate d'éthyle : 8/2), le produit attendu est isolé.

Point de fusion : 72°C.

IR(KBr) : $\nu_{C=O}$ = 1713, 1779 $cm^{-1}$ ; $\nu_{COC}$ = 1148 $cm^{-1}$.

Spectre de masse : m/z 250 (M + 1).

*Stade B : 3-[(diphénoxyphosphoryl)oxy]-4H-1,4-benzoxazine-4-carboxylate de tert-butyle*

Sous atmosphère anhydre, 12 mmol de TMEDA sont additionnés à une solution de 10 mmol du produit obtenu au stade A précédent dans 50 ml de THF anhydre. Après avoir refroidie la solution à -78°C, 12 mmol de LDA 2M (dans une solution heptane /THF) sont ajoutés goutte à goutte. Après 2 heures d'agitation 12 mmol de chlorophosphate de diphényle sont ajoutés goutte à goutte au mélange réactionnel qui est maintenue pendant 2 heures supplémentaires à -78°C. Après retour à température ambiante, la solution est hydrolysée puis extraite à l'acétate d'éthyle. La phase

organique est séchée sur sulfate de magnésium, filtrée et concentrée. Après purification du résidu par chromatographie sur gel de silice (éther de pétrole/acétate d'éthyle : 9/1), le produit attendu est isolé.
Point de fusion : 64°C.
IR(KBr) : $\nu_{C=O}$ = 1732 $cm^{-1}$; $\nu_{P=O}$ 1313 $cm^{-1}$.
Spectre de masse : m/z 482 (M + 1).

**PREPARATION B : 3-[(diphénoxyphosphoryl)oxy]-2,3-dihydro-4*H*-pyrido [3,2-b][1,4]oxazine-4-carboxylate de phényle**

*Stade A : 2,3-dihydro-4H-pyrido[3,2-b][1,4]oxazine-3-one-4-carboxylate de phényle*

Sous atmosphère anhydre, une solution de 10 mmol de 2*H*-pyrido[3,2-b][1,4]oxazin-3-one dans 50 ml de tétrahydrofurane est refroidie à -78°C. A cette température, 11 mmol d'une solution de *n*-butyllithium à 1,6M dans l'hexane sont ajoutés goutte à goutte. Après 30 minutes de temps de contact à -78°C, 11 mmol de chloroformiate de phényle sont ajoutés goutte à goutte et l'agitation est maintenue pendant 2 heures supplémentaires. Après retour à température ambiante, la solution est hydrolysée puis extraite à l'acétate d'éthyle. La phase organique est séchée sur sulfate de magnésium, filtrée et évaporée. Après purification par chromatographie sur gel de silice (éther de pétrole/acétate d'éthyle : 8/2), le produit attendu est isolé.
Point de fusion : 97°C.
IR(KBr): $\nu_{C=O}$ = 1717 $cm^{-1}$ ; 1803 $cm^{-1}$.
Spectre de masse : m/z 271 (M + 1).

*Stade B : 3-[(diphénoxyphosphoryl)oxy]-4H-pyrido[3,2-b][1,4]oxazine-4-carboxylate de phényle*

Le produit attendu est obtenu selon le procédé décrit au stade B de la préparation A à partir du composé du stade précédent.
Point de fusion : 82°C.
IR(KBr) : $\nu_{C=O}$ = 1749 $cm^{-1}$ ; $\nu_{P=O}$ 1294 $cm^{-1}$.
Spectre de masse : m/z 503 (M + 1).

**PREPARATION C : 8-(pinacolboronyl)-1,4-dioxaspiro[4.5]déc-7-ène**

*Stade A : 8-(trifluorométhyl)sulfonyloxy-1,4-dioxaspiro[4.5]déc-7-ène*

Sous atmosphère anhydre, une solution de 6,4 mmol de LDA 2M dans un mélange de THF/heptane est dilué dans 8 ml de THF. La température est abaissée à -78°C, puis 6,4 mmol de 1,4-dioxaspiro[4.5]décan-8-one en solution dans 8 ml de THF sont ajoutés lentement. Le milieu réactionnel est agité 2 heures à cette température et 9,6 mmol de N-phényltrifluorométhanesulfonimide en solution dans 8 ml de THF sont additionnés. Après agitation 15 minutes à -78°C puis retour à température ambiante pendant la nuit, le milieu est concentré. Après purification sur gel d'alumine neutre (éther de pétrole/acétate d'éthyle : 95/5), le produit attendu est isolé.
IR(film NaCl) : $\nu_{C=C}$ = 1692 $cm^{-1}$; $\nu_{SO2}$ = 1418 $cm^{-1}$.

*Stade B : 8-(pinacolboronyl)-1,4-dioxaspiro[4.5]déc-7-ène*

Sous atmosphère inerte, 0,7 mmol du produit obtenu au stade A précédent, 1,05 mmol de pinacolborane, 0,028 mmol de chlorure de bis(triphénylphosphine)palladium (II), 0,084 mmol de triphénylarsine et 2,1 mmol de triéthylamine sont mélangés dans 3 ml de toluène puis chauffé à 80°C pendant 2 heures. Après refroidissement, le résidu est repris dans l'acétate d'éthyle, lavé avec une solution saturée de chlorure de sodium. La phase organique est séchée sur sulfate de magnésium, filtrée et concentrée. Après purification du résidu par chromatographie sur gel de silice (éther de pétrole/acétate d'éthyle : 9/1), le produit attendu est isolé.
Point de fusion : 58°C.
IR(KBr) : $\nu_{C=C}$ = 1635 $cm^{-1}$; $\nu_{COC}$= 1115, 1143 $cm^{-1}$.
Spectre de masse : m/z 267 (M + 1).

**EXEMPLE 1 : 8-(tert-butoxycarbonyl)-2-[2-(diméthylamino)éthyl]-5-hydroxy-2,3-dihydrobenzo[a]pyrrolo[3,4-c]phénoxazine-1,3-dione**

*Stade A: 3-(1,4-dioxaspiro[4.5]déc-7-èn-8-yl)-4H-1,4-benzoxazine-4-carboxylate de tert-butyle*

Sous atmosphère inerte, une solution 1M de 1 mmol du produit de la préparation A et 5 % de chlorure de bis (triphénylphosphine)palladium (II) dans du tétrahydrofurane est agitée pendant 10 minutes à température ambiante 1,5 mmol du produit de la préparation C, quelques gouttes d'éthanol et 2 mmol d'une solution aqueuse de carbonate de sodium 2M sont ajoutés au mélange réactionnel qui est ensuite porté à reflux pendant une heure. Après refroidissement et hydrolyse, la solution est extraite par l'acétate d'éthyle. La phase organique est séchée sur sulfate de magnésium, filtrée et concentrée. Après purification par chromatographie sur gel de silice (éther de pétrole/acétate d'éthyle : 6/4), le produit attendu est isolé.
Point de fusion : 92-93°C.
IR(KBr) : $\nu_{C=O}$ = 1711 $cm^{-1}$ ; $\nu_{COC}$ = 1113, 1163 $cm^{-1}$.
Spectre de masse : m/z 372 (M + 1).

*Stade B : 12-(tert-butoxycarbonyl)-3,3-(1,2-éthylènedioxy)-1,2,3,4,4a,6a-hexahydro-12H-benzo[a]phénoxazine-5,6-dicarboxylate de diméthyle*

Dans un système clos, 8 mmol du produit obtenu au stade A précédent et 40 mmol d'acétylène dicarboxylate de diméthyle sont agités à 80°C pendant 22 heures. Après purification par chromatographie sur gel de silice (éther de pétrole/acétate d'éthyle : 7/3), le produit attendu est isolé.
Point de fusion : 234-235°C.
IR(KBr): $\nu_{C=O}$ = 1728 $cm^{-1}$ ; $\nu_{COC}$= 1152 $cm^{-1}$.
Spectre de masse : m/z 514 (M + 1).

*Stade C: 12-(tert-butoxycarbonyl)-3,3-(1,2-éthylènedioxy)-1,2,3,4-tétrahydro-12H-benzo[a]phénoxazine-5,6-dicarboxylate de diméthyle*

Sous atmosphère inerte, 0,92 mmol du produit obtenu au stade B précédent et 2,75 mmol de N-bromosuccinimide recristallisé sont chauffés dans 23 ml de tétrachlorure de carbone distillé pendant 10 minutes à reflux à l'aide d'une lampe de 60 W en présence d'une quantité catalytique de péroxyde de benzoyle. Après refroidissement, la solution est filtrée puis concentrée. Après purification par chromatographie sur gel de silice (éther de pétrole/acétate d'éthyle : 6/4), le produit attendu est isolé.
Point de fusion : < 50°C.
IR(KBr) : $\nu_{C=O}$ = 1701, 1717, 1733 $cm^{-1}$ ; $\nu_{COC}$ = 1152, 1195 $cm^{-1}$.
Spectre de masse : m/z 512 (M + 1).

*Stade D : 3-oxo-1,3,4,12-tétrahydro-2H-benzo[a]phénoxazine-5,6-dicarboxylate de diméthyle*

3 ml d'acide chlorhydrique 12M sont ajoutés goutte à goutte à 0,6 mmol du produit obtenu au stade C précédent dissous dans 3 ml d'éthanol. Le mélange est agité 1,5 heures à température ambiante. Après neutralisation par une solution saturée d'hydrogénocarbonate de sodium et extraction avec de l'acétate d'éthyle, la phase organique est séchée sur sulfate de magnésium, filtrée, concentrée. Après purification par chromatographie sur gel de silice (éther de pétrole/acétate d'éthyle : 5/5 à 0/10), le produit attendu est isolé.
Point de fusion : 250-251°C.
IR(KBr): $\nu_{C=O}$ = 1695, 1720 $cm^{-1}$ ; $\nu_{NH}$ = 3430 $cm^{-1}$.
Spectre de masse : m/z 366 (M + 1).

*Stade E : 12-(tert-butoxycarbonyl)-3-[(tert-butoxycarbonyl)oxy]-1,1-dihydro-12H-benzo[a]phénoxazine-5,6-dicarboxylate de diméthyle*

Sous atmosphère inerte, 0,69 mmol du composé obtenu au stade D précédent sont dissous dans 10 ml de tétrahydrofurane. Après addition de 1,73 mmol de 4-diméthylaminopyridine et 1,73 mmol de di-tert-butyldicarbonate, le milieu est laissé sous agitation pendant 12 heures. Après concentration, le résidu est repris par l'acétate d'éthyle et lavé deux fois par une solution d'acide chlorhydrique 1M. La phase organique est séchée sur sulfate de magnésium, filtrée et concentrée, permettant d'obtenir le produit attendu.
IR(film NaCl) : $\nu_{C=O}$ = 1728, 1756 $cm^{-1}$; $\nu_{COC}$= 1139 $cm^{-1}$.

Spectre de masse : m/z 568 (M + 1).

*Stade F : 12-(tert-butoxycarbonyl)-3-[(tert-butoxycarbonyl)oxy]-12H-benzo[a] phénoxazine-5,6-dicarboxylate de diméthyle*

Sous atmosphère inerte 0,62 mmol du composé obtenu au stade E précédent sont dissous dans 5 ml de toluène en présence de 4,96 mmol de 2,3-dichloro-5,6-dicyano-1,4-benzoquinone et le milieu est chauffé à 90°C pendant 24 heures. Après refroidissement et concentration, le milieu réactionnel est repris par du dichlorométhane et lavé par une solution de soude à 8 %. La phase aqueuse est extraite par le dichlorométhane et les phases organiques sont rassemblées, séchées sur sulfate de magnésium, filtrées et concentrées. Après purification par chromatographie su gel silice (éther de pétrole/acétate d'éthyle : 7/3), le produit attendu est isolé.
Point de fusion : 101-102°C.
IR(KBr): $\nu_{C=O}$ = 1731, 1739, 1756, 1766 $cm^{-1}$; $\nu_{COC}$ = 1149 $cm^{-1}$.
Spectre de masse : m/z 566 (M + 1).

*Stade G : 12-(tert-butoxycarbonyl)-3-hydroxy-12H-benzo[a]phénoxazine-5,6-dicarboxylate de diméthyle*

Sous atmosphère inerte, 0,28 mmol du composé obtenu au stade F précédent sont dissous dans 2 ml de méthanol en présence de 0,34 mmol de méthylate de sodium. Le milieu est laissé sous agitation à température ambiante pendant 12 heures. Après concentration et hydrolyse, le milieu est extrait par l'acétate d'éthyle, séché sur sulfate de magnésium, filtré et évaporé. Après purification par chromatographie sur gel silice (éther de pétrole/acétate d'éthyle : 7/3), le produit attendu est isolé.
Point de fusion : 90-91°C (décomposition).
IR(KBr): $\nu_{C=O}$ = 1722 $cm^{-1}$; $\nu_{COC}$ = 1152 $cm^{-1}$ ; $\nu_{OH}$ = 3442 $cm^{-1}$.
Spectre de masse : m/z 466 (M + 1).

*Stade H: 8-(tert-butoxycarbonyl)-2-[2-(diméthylamino)éthyl]-5-hydroxy-2,3-dihydrobenzo[a]pyrrolo[3,4-c]phénoxazine-1,3-dione*

Sous atmosphère inerte, 0,26 mmol du composé obtenu au stade G précédent sont chauffés à 100°C dans 4 ml de N,N-diméthyléthylène diamine pendant 7 heures. Après refroidissement l'excès de diamine est évaporé. Après purification par chromatographie sur gel de silice (dichlorométhane/méthanol : 95/5), le produit attendu est isolé.
Point de fusion : 190°C (dégradation).
IR(KBr): $\nu_{C=O}$ = 1705, 1762 $cm^{-1}$ ; $\nu_{CO}$ = 1249 $cm^{-1}$ ; $\nu_{OH}$ = 3446 $cm^{-1}$.
Spectre de masse : m/z 490,5 (M + 1).

**EXEMPLE 2 : chlorhydrate de 2-[2-(diméthylamino)éthyl]-5-hydroxybenzo[a] pyrrolo[3,4-c]phénoxazine-1,3-dione**

3 ml d'acide chlorhydrique 12M sont ajoutés goutte à goutte à 0,2 mmol du composé de l'exemple 1 dissous dans 4 ml d'éthanol. Le mélange réactionnel est agité 1,5 heures à température ambiante puis concentré. Par addition d'éther éthylique, il se forme un précipité qui est filtré, permettant d'obtenir le produit attendu.
IR(KBr): $\nu_{C=O}$ = 1686, 1744 $cm^{-1}$ ; $\nu_{NH, OH}$ = 3431 $cm^{-1}$.
Spectre de masse : m/z 390 (M + 1).

**EXEMPLE 3 : 8-(tert-butoxycarbonyl)-2-[2-(diméthylamino)éthyl]-5-(2-hydroxyéthoxy)-2,3-dihydrobenzo[a] pyrrolo[3,4-c] phénoxazine-1,3-dione**

*Stade A : 12-(tert-butoxycarbonyl)-3-(2-hydroxyéthoxy)-12H benzo[a]phénoxazine-5,6-dicarboxylate de diméthyle*

Le produit attendu est obtenu selon le procédé décrit au stade F de l'exemple 1 à partir du composé du stade B de l'exemple 1.
Point de fusion : 87-88°C.
IR(KBr) : $\nu_{C=O}$ = 1725$cm^{-1}$ ; $\nu_{OH}$ = 3440 $cm^{-1}$.
Spectre de masse : m/z 510 (M + 1).

*Stade B : 8-(tert-butoxycarbonyl)-2-[2-(diméthylamino)éthyl]-5-(2-hydroxyéthoxy)- 2,3-dihydrobenzo[a]pyrrolo[3,4-c] phénoxazine-1,3-dione*

Le produit attendu est obtenu selon le procédé décrit au stade H de l'exemple 1 à partir du composé du stade A précédent.
Point de fusion : > 80°C (dégradation).
IR(KBr) : $\nu_{C=O}$ = 1707, 1763 $cm^{-1}$ ; $\nu_{OH}$ = 3447 $cm^{-1}$ .
Spectre de masse : m/z 534 (M + 1).

**EXEMPLE 4 : 8-(tert-butoxycarbonyl)-2-[2-(diméthylamino)éthyl]-5{2-[(méthylsulfonyl)oxy]éthoxy}-2,3-dihydrobenzo[a]pyrrolo [3,4-c]phénoxazine-1,3-dione**

Sous atmosphère inerte, 0,93 mmol de triéthylamine puis 0,93 mmol de chlorure de mésyle sont ajoutés à une solution de 0,06 mmol du composé de l'exemple 3 dans 3 ml de dichlorométhane à 0°C. L'agitation est maintenue à 0°C pendant 8 heures. A température ambiante, la solution est hydrolysée puis extraite par le dichlorométhane. La phase organique est séchée sur sulfate de magnésium, filtrée et concentrée. Après purification par chromatographie sur gel de silice (dichlorométhane/méthanol : 95/5), le produit attendu est isolé.
Point de fusion : 70-80°C (gomme).
IR(KBr) : $\nu_{C=O}$ = 1707, 1763 $cm^{-1}$.
Spectre de masse : m/z 612 (M + 1).

**EXEMPLE 5 : 2-[2-(diméthylamino)éthyl]-5-{2-[(méthylsulfonyl)oxy]éthoxy}-1,2,3,8-tétrahydrobenzo[a]pyrrolo [3,4-c]phénoxazine-1,3-dione**

0,03 mmol du composé de l'exemple 4 sont dissous dans 1 ml d'acide formique et laissé sous agitation à température ambiante pendant 3 heures. Après concentration, le résidu est repris par le dichlorométhane et lavé avec une solution de carbonate de sodium 2M puis avec de l'eau. Les phases organiques sont rassemblées, séchées sur sulfate de magnésium, filtrées, concentrées. Après purification par chromatographie sur gel de silice (dichlorométhane/méthanol : 9/1), le produit attendu est isolé.
IR(KBr) : $\nu_{C=O}$ = 1686, 1702 $cm^{-1}$; $\nu_{NH}$ = 3432 $cm^{-1}$.
Spectre de masse : m/z 512 (M + 1).

**EXEMPLE 6 : 2-[2-(diméthylamino)éthyl]-5-(2-hydroxyéthoxy)benzo[a]pyrrolo [3,4-c]phénoxazine-1,3-dione**

Le produit attendu est obtenu selon le procédé de l'exemple 5 à partir du composé de l'exemple 3.
Point de fusion : 216°C (gomme).
IR(KBr): $\nu_{C=O}$ = 1690, 1741 $cm^{-1}$; $\nu_{NH}$ = 3427 $cm^{-1}$.
Spectre de masse : m/z 434 (M + 1).

**EXEMPLE 7 : 8-(formyl)-2-[2-(diméthylamino)éthyl]-5-(2-hydroxyéthoxy)benzo [a]pyrrolo[3,4-c]phénoxazine-1,3-dione**

Le produit attendu est obtenu lors de la purification par chromatographie sur gel de silice de l'exemple 6.
Point de fusion : 202°C.
IR(KBr) : $\nu_{C=O}$ = 1693, 1732 $cm^{-1}$ ; $\nu_{NH}$ = 3428 $cm^{-1}$.
Spectre de masse : m/z 462 (M + 1).

**EXEMPLE 8 : 8-(phénoxycarbonyl)-5-(2-hydroxyéthoxy)-2-méthyl-2,3-dihydrobenzo[e]pyrido[2',3':5,6][1,4] oxazino[3,2-g]isoindole-1,3-**dione

*StadeA : 3-(1,4-dioxaspiro[4.5]déc-7-èn-8-yl)-4H-pyrido[3,2-b][1,4]oxazine-4-carboxylate de phényle*

Le produit attendu est obtenu selon le procédé du stade A de l'exemple 1 à partir du composé de la préparation B.
IR(KBr) : $\nu_{C=O}$ = 1741 $cm^{-1}$; $\nu_{COC}$ = 1111, 1197 $cm^{-1}$.
Spectre de masse : m/z 393 (M + 1).

*Stade B: 8-(phénoxycarbonyl)-5,5-(1,2-éthylènedioxy)-2-méthyl-2,3,3a,3b,4,5,6,7, 13a,13b-décahydrobenzo[e]pyrido [2',3':5,6][1,4]oxazino[3,2-g]isoindole -1,3-dione*

Dans un système clos, 1 mmol du produit obtenu au stade A précédent et 3 mmol de N-méthylmaléimide sont agités à 95°C pendant 2 heures en présence de quelques gouttes de toluène. Après purification par chromatographie sur gel de silice (éther de pétrole/acétate d'éthyle : 6/4), le produit est isolé.
Point de fusion : 150°C (gomme).
IR(KBr) : $\nu_{C=O}$ = 1701, 1786 $cm^{-1}$.
Spectre de masse : m/z 504 (M + 1).

*Stade C : 8-(phénoxycarbonyl)-5-(2-hydroxyéthoxy)-2-méthyl-2,3-dihydrobenzo [e]pyrido[2',3':5,6][1,4]oxazino[3,2-g] isoindole-1,3-dione*

Le produit attendu est obtenu selon le procédé décrit au stade C de l'exemple 1 à partir du composé du stade B précédent.
Point de fusion : 250°C (gomme).
IR(KBr): $\nu_{C=O}$ = 1707, 1752 $cm^{-1}$; $\nu_{COC}$ = 1191 $cm^{-1}$; $\nu_{OH}$ = 3463 $cm^{-1}$.
Spectre de masse : m/z 498 (M + 1).

**EXEMPLE 9 : 2-[2-(diméthylamino)éthyl]-5-(2-hydroxyétyhoxy)benzo[e]pyrido [2',3':5,6][1,4]oxazino[3,2-g] isoindole-1,3-dione**

Le produit attendu est obtenu selon le procédé décrit au stade H de l'exemple 1 à partir du composé de l'exemple 8.
Spectre de masse : m/z 435 (M + 1).

**EXEMPLE 10 : 8-(tert-butoxycarbonyl)-2-[2-(diéthylamino)éthyl]-5-hydroxy-2,3-dihydrobenzo[a]pyrrolo[3,4-c] phénoxazine-1,3-dione**

Le produit attendu est obtenu selon le procédé décrit au stade H de l'exemple 1 à partir du composé du stade G de l'exemple 1 et de N,N-diéthyléthylène diamine.

**EXEMPLE 11 : 2-[2-(diéthylamino)éthyl]-5-hydroxybenzo[a]pyrrolo[3,4-c] phénoxazine-1,3-dione**

Le produit attendu est obtenu selon le procédé de l'exemple 2 à partir du composé de l'exemple 10.

***ETUDE PHARMACOLOGIOUE DES COMPOSES DE L'INVENTION***

**EXEMPLE 12 : Activité in vitro**

✧ Leucémie murine L1210

La leucémie murine L1210 a été utilisée in vitro. Les cellules sont cultivées dans du milieu de culture RPMI 1640 complet contenant 10 % de sérum de veau foetal, 2 mM de glutamine, 50 U/ml de pénicilline, 50 μg/ml de streptomycine et 10 mM d'Hepes, pH : 7,4. Les cellules sont réparties dans des microplaques et exposées aux composés cytotoxiques pendant 4 temps de doublement, soit 48 heures. Le nombre de cellules viables est ensuite quantifié par un essai colorimétrique, le Microculture Tetrazolium Assay (J. Carmichael et al., Cancer Res.; 47, 936-942, (1987)). Les résultats sont exprimés en $IC_{50}$, concentration en cytotoxique qui inhibe à 50 % la prolifération des cellules traitées. Tous les produits de l'invention montrent une bonne cytotoxicité sur cette lignée cellulaire. A Titre d'exemple, le composé de l'exemple 2 présente une $IC_{50}$ de 0,25 μM sur L1210.

✧ Lignées cellulaires humaines

Les composés de l'invention ont également été testés sur des lignées cellulaires humaines issues de tumeurs solides selon le même protocole expérimental que celui décrit sur la leucémie murine L1210 mais avec des temps d'incubation de 4 jours au lieux de 2 jours. A titre indicatif, le composé de l'exemple 2 présente un $IC_{50}$ de 0,27 μM sur le carcinome de la prostate DU145, 0,16 μM sur le carcinome pulmonaire non à petites cellules A549, 0,6 μM sur le carcinome du colon HT-29 et 0,26 μM sur le carcinome épidermoïde KB-3-1.

Ces différents résultats démontrent clairement le fort potentiel anti-tumoral des composés de l'invention, sur les leucémie et les tumeurs solides.

**EXEMPLE 13 : Action sur le cycle cellulaire**

Les cellules L1210 sont incubées pendant 21 heures à 37°C en présence de différentes concentrations en produit testés. Les cellules sont ensuite fixées par de l'éthanol à 70 % (v/v), lavées deux fois dans du PBS et incubées 30 minutes à 20°C dans du PBS contenant 100 μg/ml de RNAse et 50 μg/ml d'iodure de propidium. Les résultats sont exprimés en pourcentage des cellules accumulées en phase G2+M après 21 heures par rapport au témoin (témoin : 20 %). Les composés de l'invention sont particulièrement intéressants. Ainsi, ils induisent une accumulation d'au moins 80 % des cellules en phase G2 + M après 21 heures à une concentration inférieure à 2,5 μM.

**EXEMPLE 14 : Activité in vivo**

***Activité antitumorale sur la leucémie P 388***

La lignée P388 (leucémie murine) a été fournie par le National Cancer Institute (Frederick, USA). Les cellules tumorales ($10^6$ cellules) ont été inoculées au jour 0 dans la cavité péritonéale de souris B6D2F1 femelles (Iffa Credo, France). Six souris de 18 à 20 g ont été utilisées par groupe expérimental. Les produits ont été administrés par voie intrapéritonéale au jour 1.
L'activité antitumorale est exprimée en % de T/C :

$$\text{T/C \% (souris)} = \frac{\text{Temps de survie médian des animaux traités}}{\text{Temps de survie médian des animaux contrôles}} \times 100$$

Les résultats obtenus montrent une excellente activité in vivo sur le modèle leucémique P 388 avec un T/C de 210 % pour une dose de 50 mg/kg, ainsi qu'une faible toxicité des composés témoin d'un excellent index thérapeutique.

**EXEMPLE 15 : Composition pharmaceutique : soluté injectable**

| | |
|---|---|
| Composé de l'exemple 2 | 10 mg |
| Eau distillée pour préparations injectables | 25 ml |

## Revendications

1. Composés de formule (I) :

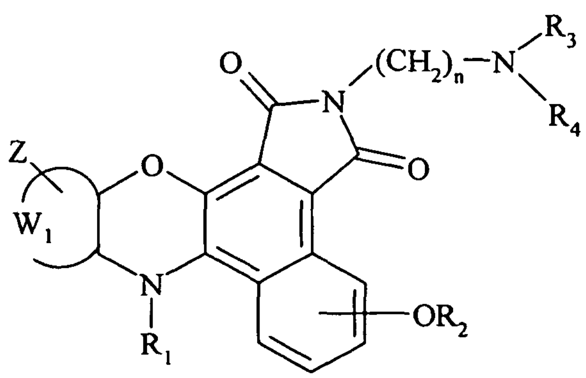

(I)

dans laquelle :

• **$W_1$** représente avec les atomes de carbone auxquels il est lié, un groupement phényle ou un groupement pyridinyle,
• **Z** représente un atome d'hydrogène,
• **$R_1$** représente un groupement choisi parmi atome d'hydrogène, -C(O)-$R_5$,
dans lesquels :

⇒ $R_5$ représente un groupement choisi parmi atome d'hydrogène, alkoxy ($C_1$-$C_6$) linéaire ou ramifié, ou aryloxy,

• **$R_2$** représente un atome d'hydrogène ou un groupement de formule -$CH_2CH_2O$-$R_8$ dans laquelle :

$R_8$ représente un groupement choisi parmi atome d'hydrogène, -$S(O)_t$-$R_6$ (dans lequel $R_6$ représente un groupement alkyle ($C_1$-$C_6$) linéaire ou ramifié t représente 2,
**$R_3$, $R_4$,** identiques ou différents, indépendamment l'un de l'autre, représentent un groupement alkyle ($C_1$-$C_6$) linéaire ou ramifié,

• n représente un entier compris entre 1 et 6 inclus

leurs énantiomères, diastéréoisomères, N-oxyde, ainsi que leurs sels d'addition à un acide ou à une base pharmaceutiquement acceptable,
étant entendu que par aryle, on comprend un groupement phényle, naphtyle, dihydronaphtyle, tétrahydronaphtyle, indényle ou indanyle, chacun de ces groupements étant éventuellement substitué par un ou plusieurs groupements, identiques ou différents, choisis parmi halogène, alkyle ($C_1$-$C_6$) linéaire ou ramifié, triahalogénoalkyle ($C_1$-$C_6$) linéaire ou ramifié, hydroxy, alkoxy ($C_1$-$C_6$) linéaire ou ramifié, et amino éventuellement substitué par un ou deux groupements alkyle ($C_1$-$C_6$) linéaire ou ramifié.

2. Composé de formule (I) selon la revendication 1, **caractérisés en ce qu'**ils représentent des composés de formule (IA) :

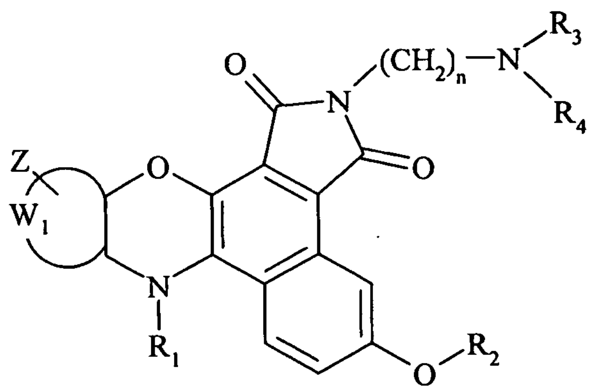

(IA)

dans laquelle $R_1$, $R_2$, $R_3$, $R_4$, $W_1$, Z et n sont tels que définis dans la formule (I), leurs énantiomères, diastéréoisomères, N-oxyde, ainsi que leurs sels d'addition à un acide ou à une base pharmaceutiquement acceptable.

3. Composés de formule (I) selon l'une quelconque des revendications 1 ou 2, **caractérisés en ce qu'**ils représentent des composés de formule (IB) :

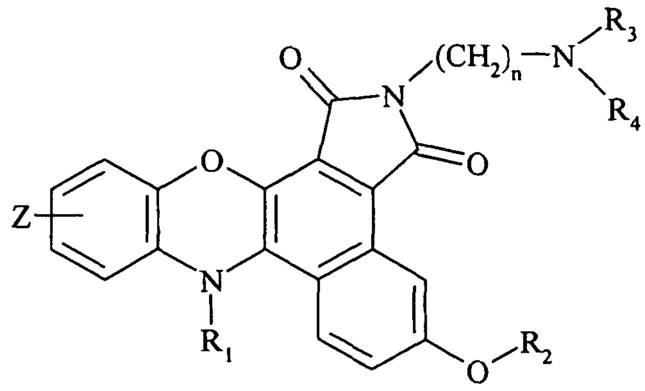

(IB)

.

dans laquelle $R_1$, $R_2$, $R_3$, $R_4$, Z et n sont tels que définis précédemment, leurs énantiomères, diastéréoisomères, N-oxyde, ainsi que leurs sels d'addition à un acide ou à une base pharmaceutiquement acceptable.

**4.** Composés de formule (I) selon l'une quelconque des revendications 1 ou 2, **caractérisés en ce qu'**ils représentent des composés de formule (IC) :

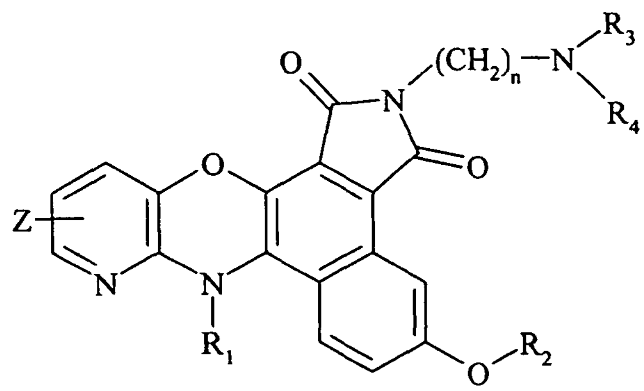

(IC)

dans laquelle $R_1$, $R_2$, $R_3$, $R_4$, Z et n sont tels que définis précédemment, leurs énantiomères, diastéréoisomères, N-oxyde, ainsi que leurs sels d'addition à un acide ou à une base pharmaceutiquement acceptable.

**5.** Composés de formule (I) selon l'une quelconque des revendications 1 à 4, **caractérisés en ce que** $R_1$ représente un atome d'hydrogène ou un groupement -C(O)-$R_5$ dans lequel $R_5$ représente plus particulièrement un atome d'hydrogène, leurs énantiomères, diastéréoisomères, N-oxyde, ainsi que leurs sels d'addition à un acide ou à une base pharmaceutiquement acceptable.

**6.** Composés de formule (I) selon l'une quelconque des revendications 1 à 5, **caractérisés en ce que** $R_2$ représente un atome d'hydrogène ou un groupement $-CH_2CH_2O-R_8$ dans lequel $R_8$ représente plus particulièrement un atome d'hydrogène, leurs énantiomères, diastéréoisomères, N-oxyde, ainsi que leurs sels d'addition à un acide ou à une base pharmaceutiquement acceptable.

**7.** Composés de formule (I) selon l'une quelconque des revendications 1 à 6, **caractérisés en ce que** n représente un nombre entier 2, leurs énantiomères, diastéréoisomères, N-oxyde, ainsi que leurs sels d'addition à un acide ou à une base pharmaceutiquement acceptable.

**8.** Composés de formule (I) selon la revendication 1 qui sont le :

- 2-[2-(diméthylamino)éthyl]-5-hydroxybenzo[a]pyrrolo[3,4-c]phénoxazine-1,3-dione,
- 2-[2-(diéthylamino)éthyl]-5-hydroxybenzo[a]pyrrolo[3,4-c]phénoxazine-1,3-dione,
- 2-[2-(diméthylamino)éthyl]-5-(2-hydroxyéthoxy)-2,3-dihydrobenzo[a]pyrrolo [3,4-c]phénoxazine-8-carbaldéhyde-1,3-dione,

- 2-[2-(diméthylarnino)éthyl]-5-(2-hydroxyéthoxy)benzo[a]pyrrolo[3,4-c] phénoxazine-1,3-dione,
- 2-[2-(diméthylamino)éthyl]-5-{2-[(méthylsulfonyl)oxy]éthoxy}-1,2,3,8-tétrahydrobenzo[a]pyrrolo[3,4-c]phénoxazine-1,3-dione
- 2-[2-(diméthylamino)éthyl]-5-(2-hydroxyéthoxy)benzo[e]pyrido[2',3':5,6][1,4]-oxazino[3,2-g]isoindole-1,3-dione,

leurs énantiomères, diastéréoisomères, N-oxyde, ainsi que leurs sels d'addition à un acide ou à une base pharmaceutiquement acceptable.

9. Procédé de préparation des composés de formule (I) selon la revendication 1, **caractérisé en ce que** l'on utilise comme produit de départ un composé de formule (II) :

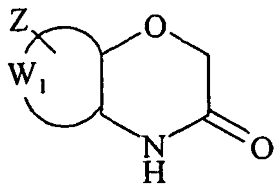

(II)

dans laquelle $W_1$ et Z sont tels que définis dans la formule (I),
composé de formule (II) dont on protège la fonction amine avec un groupement protecteur $P_G$ bien connu de l'homme de l'art pour conduire au composé de formule (III) :

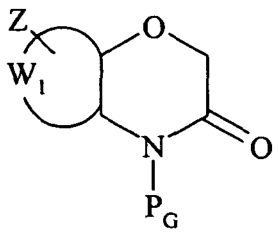

(III)

dans laquelle $P_G$ représente un groupement tert-butyloxycarbonyl ou phénoxycarbonyl et $W_1$ et Z sont tels que définis précédemment,
composé de formule (III) qui est traité par du diisopropylamidure de lithium suivi de chlorophosphate de diphényle pour conduire au composé de formule (IV) :

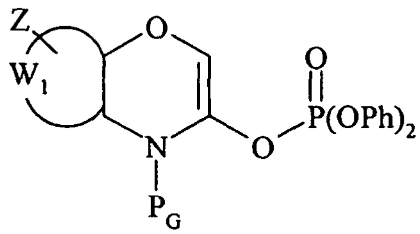

(IV)

dans laquelle $P_G$, $W_1$, et Z sont tels que définis précédemment,
composé de formule (IV) qui est traité, en présence de chlorure de bis(triphénylphosphine)palladium, par un composé de formule (V) :

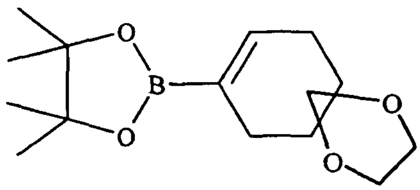

(V)

pour conduire au composé de formule (VI) :

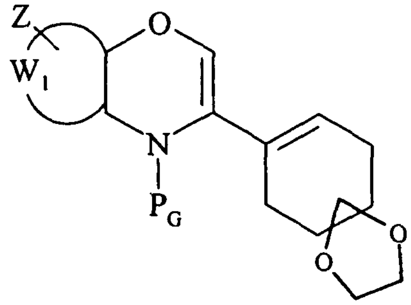

(VI)

dans laquelle $P_G$, $W_1$ et Z sont tels que définis précédemment,
composé de formule (VI) qui est :

- soit traité sous atmosphère inerte, par de l'acétylène dicarboxylate de diméthyle, pour conduire au composé de formule (VII) :

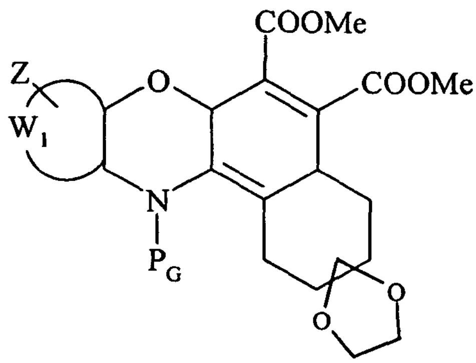

(VII)

dans laquelle $P_G$, $W_1$ et Z sont tels que définis précédemment,
composé de formule (VII) qui est :

♦ soit mis en présence de N-bromosuccinimide et de peroxyde de benzoyle, pour conduire au composé de formule (VIII) :

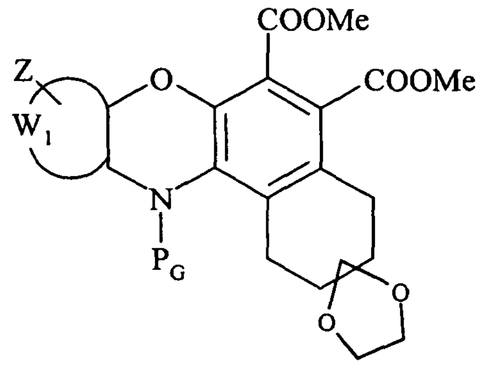

(VIII)

dans laquelle $P_G$, $W_1$ et Z sont tels que définis précédemment, composé de formule (VIII) qui est soumis à l'action d'acide chlorhydrique pour conduire au composé de formule (IX) :

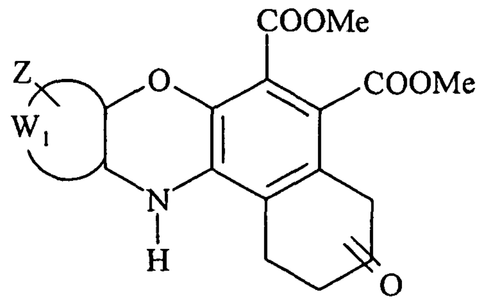

(IX)

dans laquelle $W_1$ et Z sont tels que définis précédemment, composé de formule (IX) qui est soumis à l'action de di-tert-butyldicarbonate en présence de 4-diméthylaminopyridine, pour conduire au composé de formule (X) :

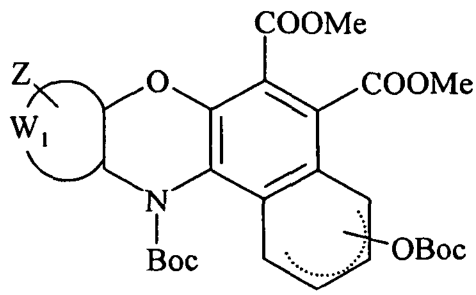

(X)

dans laquelle  représente une liaison simple ou double, Boc représente un groupement tert-butyloxycarbonyl et $W_1$ et Z sont tels que définis précédemment, composé de formule (X) qui est soumis à l'action de 2,3-dichloro-5,6-dicyano-1,4-benzoquinone, pour conduire au composé de formule (XI) :

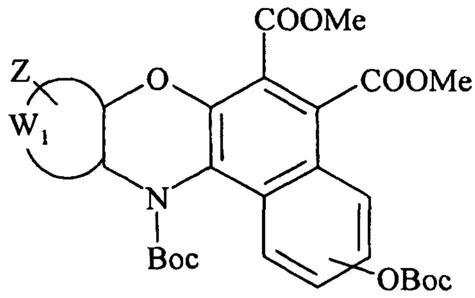

(XI)

dans laquelle Boc, $W_1$ et Z sont tels que définis précédemment,
composé de formule (XI) qui est soumis à l'action de méthanolate de sodium puis qui est hydrolysé pour conduire au composé de formule (XII) :

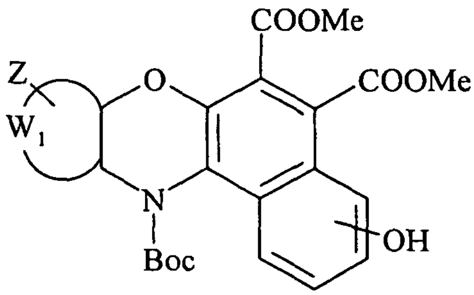

(XII)

dans laquelle Boc, $W_1$ et Z sont tels que définis précédemment,
composé de formule (XII) qui est soumis à l'action d'un composé de formule (XIII) :

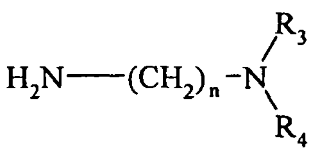

(XIII)

dans laquelle $R_3$, $R_4$ et n sont tels que définis dans la formule (I), pour conduire au composés de formule (I/a), cas particulier des composés de formule (I) :

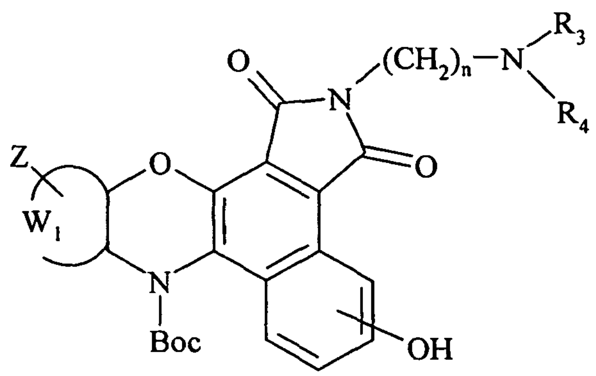

(I/a)

dans laquelle Boc, $R_3$, $R_4$, $W_1$, Z et n sont tels que définis précédemment,
composé de formule (I/a) qui est éventuellement soumis aux mêmes conditions de réaction que le composé de formule (VIII), pour conduire au composé de formule (I/b), cas particulier des composés de formule (I) :

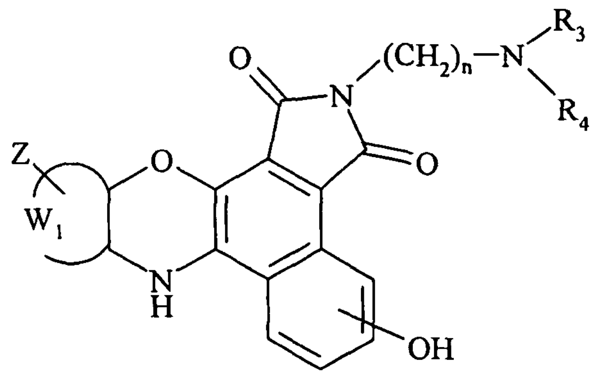

(I/b)

dans laquelle $R_3$, $R_4$, $W_1$, Z et n sont tels que définis précédemment,

♦ soit soumis aux mêmes conditions de réaction que le composé de formule (X), pour conduire au composé de formule (XIV) :

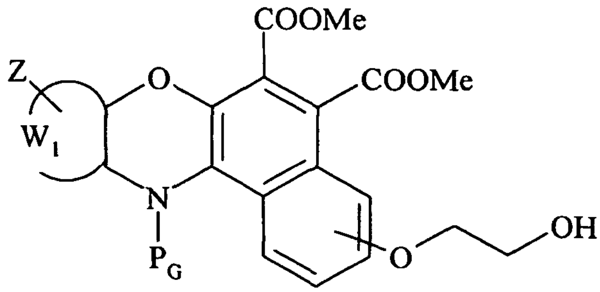

(XIV)

dans laquelle $P_G$, $W_1$, et Z sont tels que définis précédemment,

composé de formule (XIV) qui est soumis aux mêmes conditions de réaction que le composé de formule (XII), pour conduire au composé de formule (I/c), cas particulier des composés de formule (I) :

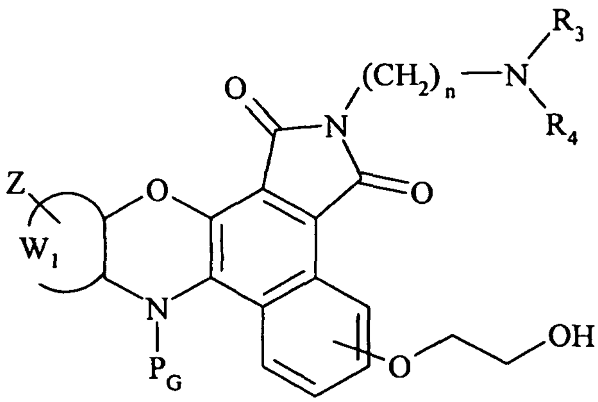

(I/c)

dans laquelle $P_G$, $R_3$, $R_4$, $W_1$, Z et n sont tels que définis précédemment, composés de formule (I/c) qui est :

■ soit éventuellement soumis à l'action d'acide formique pour conduire aux composés de formule (I/d) et (I/e), cas particuliers des composés de formule (I) :

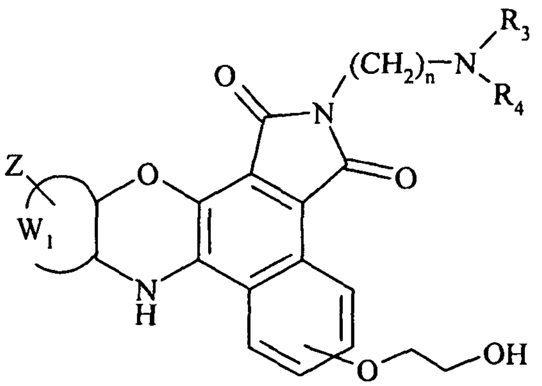

(I/d)

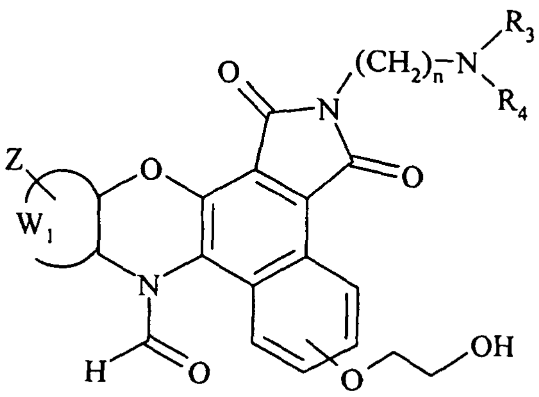

(I/e)

dans laquelle $R_3$, $R_4$, $W_1$, Z et n sont tels que définis précédemment,

■ soit éventuellement soumis à l'action d'un composé de formule (XV) :

$R_{8a}$-G (XV)

dans laquelle G représente un groupement partant et $R_{8a}$, différent de atome d'hydrogène, prend la même définition que $R_8$ dans la formule (I), pour conduire au composé de formule (I/f), cas particulier des composés de formule (I),

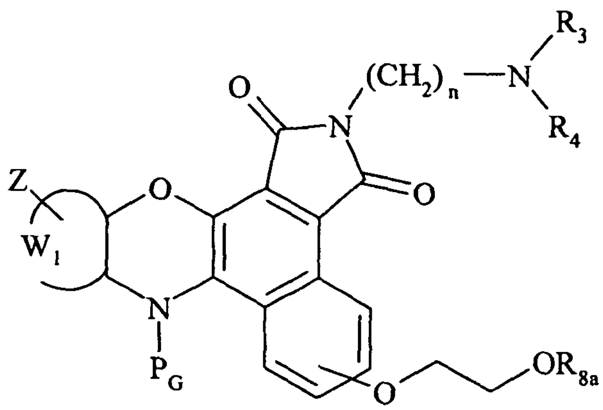

(I/f)

dans laquelle $P_G$, $R_3$, $R_4$, $R_{8a}$, $W_1$, Z et n sont tels que définis précédemment, composés de formule (I/f) dont on déprotège éventuellement la fonction amine selon des méthodes classiques de la synthèse organique pour conduire au composé de formule (I/g), cas particulier des composés de formule (I):

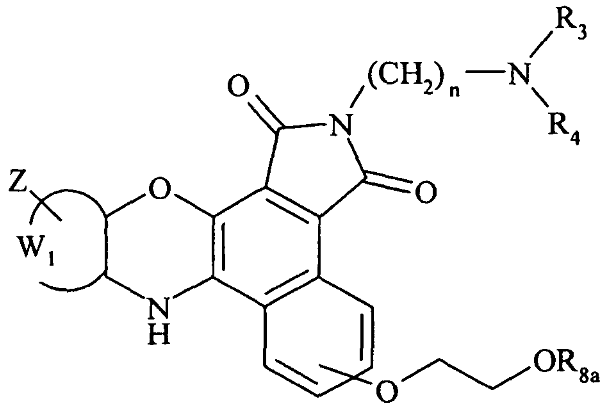

(I/g)

dans laquelle $R_3$, $R_4$, $R_{8a}$, $W_1$, Z et n sont tels que définis précédemment, les composés de formule (I/b), (I/d) et (I/g) forment les composés de formule (I/h) :

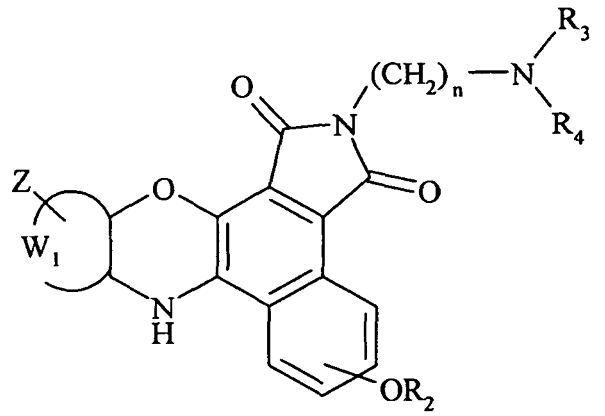

(I/h)

dans laquelle $R_2$, $R_3$, $R_4$, $W_1$, Z et n sont tels que définis précédemment, composés de formule (I/h) qui sont éventuellement soumis à l'action d'un composé de formule (XVI) :

$R_{1a}$-G (XVI)

dans laquelle $R_{1a}$, différent de atome d'hydrogène, prend la même définition que $R_1$ dans la formule (I) et G est tel que défini précédemment, pour conduire au composé de formule (I/i), cas particulier des composés de formule (I) :

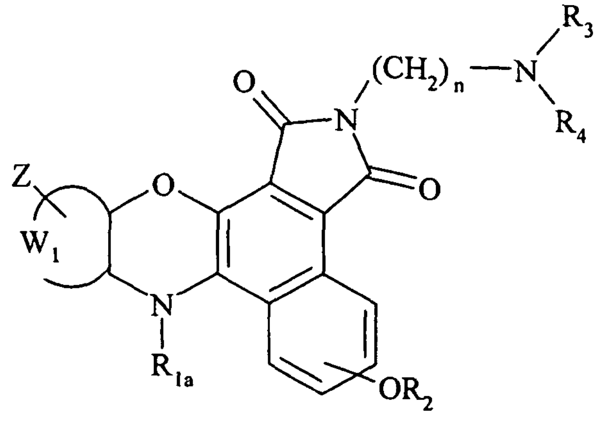

(I/i)

dans laquelle $R_{1a}$, $R_2$, $R_3$, $R_4$, $W_1$, Z et n sont tels que définis précédemment,

• soit traité avec du N-méthylmaléimide, pour conduire au composé de formule (XVII) :

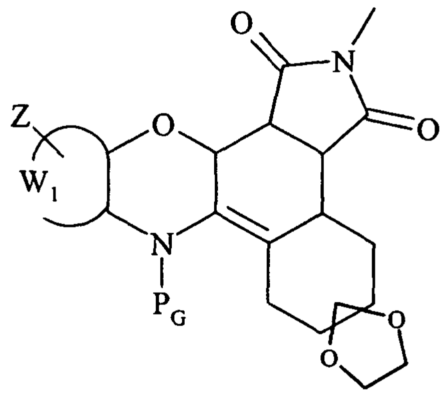

(XVII)

dans laquelle $P_G$, $W_1$ et Z sont tels que définis précédemment,
composé de formule (XVII) qui est soumis aux mêmes conditions de réaction que le composé de formule (VII) pour conduire au composé de formule (XVIII) :

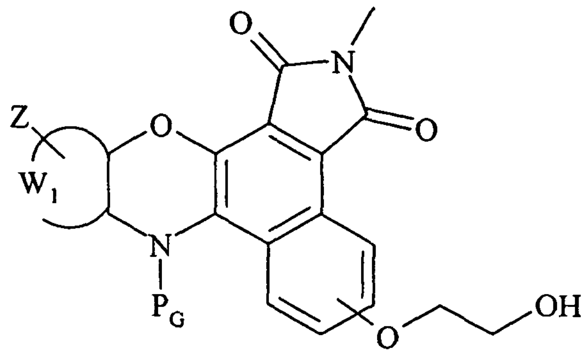

(XVIII)

dans laquelle $P_G$, $W_1$, et Z sont tels que définis précédemment,
composé de formule (XVIII) qui est soumis aux mêmes conditions de réactions que le composé de formule (XII) pour conduire au composé de formule (I/d) tel que décrit précédemment,
les composés de formule (I/a) à (I/i) forment l'ensemble des composés de formule (I), que l'on purifie le cas échéant, selon des techniques classiques de purification, qui peuvent si on le désire, être séparés en leurs différents isomères, selon une technique classique de séparation, et que l'on transforme, si on le souhaite, en leurs N-oxydes et, le cas échéant, en leurs sels d'addition à un acide ou à une base pharmaceutiquement acceptable.

**10.** Compositions pharmaceutiques contenant comme principe actif au moins un composé de formule (I), selon l'une quelconque des revendications 1 à 8, seul ou en combinaison avec un ou plusieurs excipients ou véhicules inertes, non toxiques, pharmaceutiquement acceptables.

**11.** Compositions pharmaceutiques selon la revendication 10, utile en tant que médicament, dans le traitement des cancers.

**12.** Composés de formule (X), (XI) et (XIV) :

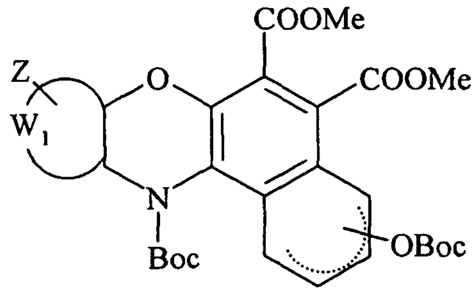

(X)

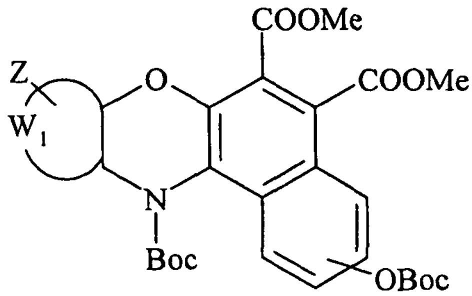

(XI)

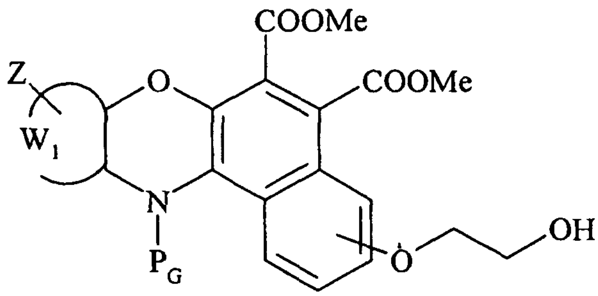

(XIV)

utiles en tant qu'intermédiaires de synthèse des composés de formule (I).

## Claims

1. Compounds of formula (I):

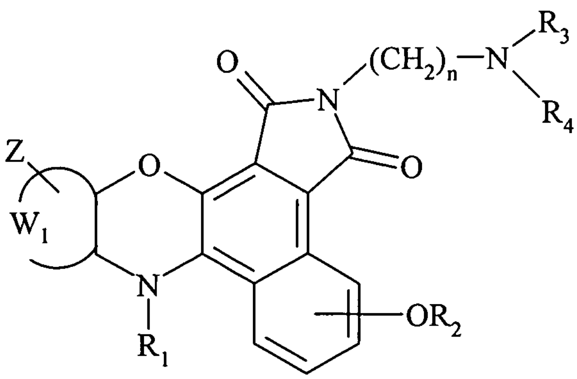

(I)

wherein :

- **$W_1$** represents, with the carbon atoms to which it is attached, a phenyl group or a pyridyl group,
- **Z** represents a hydrogen atom,
- **$R_1$** represents a group selected from hydrogen and -C(O)-$R_5$,

wherein:

⇒ $R_5$ represents a group selected from hydrogen and the groups linear or branched ($C_1$-$C_6$)alkoxy and aryloxy,

- **$R_2$** represents a hydrogen atom or a group of formula -$CH_2CH_2O$-$R_8$ wherein :

$R_8$ represents a group selected from hydrogen and -$S(O)_t$-$R_6$ (wherein $R_6$ represents a linear or branched ($C_1$-$C_6$)alkyl group and t represents 2),
**$R_3$** and **$R_4$,** which may be identical or different, each represents, independently of the other, a linear or branched ($C_1$-$C_6$)alkyl group,

- n represents an integer of from 1 to 6 inclusive, their enantiomers, diastereoisomers, N-oxides, and addition salts thereof with a pharmaceutically acceptable acid or base,

wherein "aryl" is to be understood as meaning a phenyl, naphthyl, dihydronaphthyl, tetrahydronaphthyl, indenyl or indanyl group, each of those groups being optionally substituted by one or more identical or different groups selected from halogen, linear or branched ($C_1$-$C_6$)alkyl, linear or branched ($C_1$-$C_6$)trihaloalkyl, hydroxy, linear or branched ($C_1$-$C_6$)alkoxy, and amino optionally substituted by one or two linear or branched ($C_1$-$C_6$)alkyl groups.

**2.** Compound of formula (I) according to claim 1, **characterised in that** they represent compounds of formula (IA) :

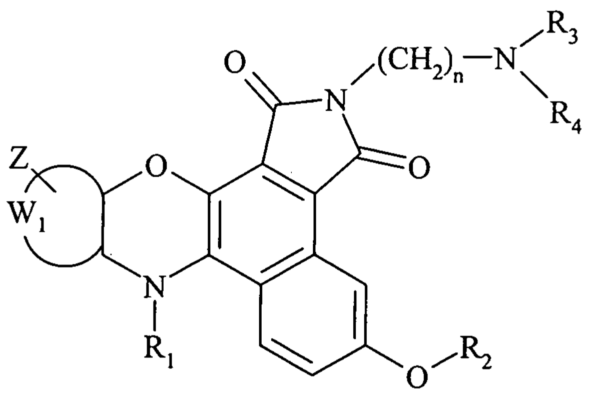

(IA)

wherein $R_1$, $R_2$, $R_3$, $R_4$, $W_1$, Z and n are as defined for formula (I), their enantiomers, diastereoisomers, N-oxides, and addition salts thereof with a pharmaceutically acceptable acid or base.

**3.** Compounds of formula (I) according to either claim 1 or claim 2, **characterised in that** they represent compounds of formula (IB) :

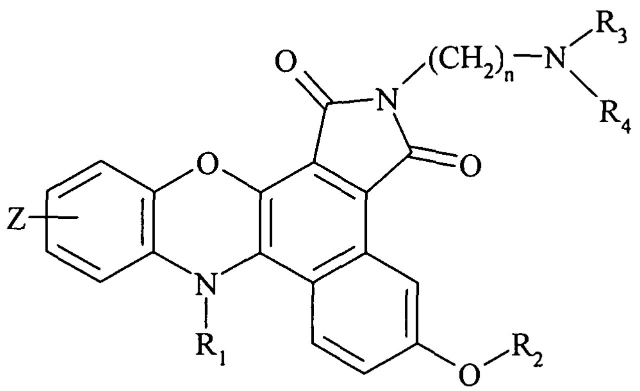

(IB)

wherein $R_1$, $R_2$, $R_3$, $R_4$, Z and n are as defined hereinbefore, their enantiomers, diastereoisomers, N-oxides, and addition salts thereof with a pharmaceutically acceptable acid or base.

**4.** Compounds of formula (I) according to either claim 1 or claim 2, **characterised in that** they represent compounds of formula (IC) :

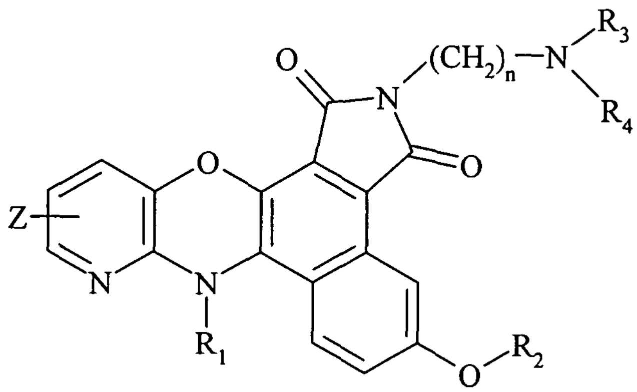

(IC)

wherein $R_1$, $R_2$, $R_3$, $R_4$, Z and n are as defined hereinbefore, their enantiomers, diastereoisomers, N-oxides, and addition salts thereof with a pharmaceutically acceptable acid or base.

**5.** Compounds of formula (I) according to any one of claims 1 to 4, **characterised in that** $R_1$ represents a hydrogen atom or a -C(O)-$R_5$ group wherein $R_5$ represents more especially a hydrogen atom, their enantiomers, diastereoisomers, N-oxides, and addition salts thereof with a pharmaceutically acceptable acid or base.

**6.** Compounds of formula (I) according to any one of claims 1 to 5, **characterised in that** $R_2$ represents a hydrogen atom or a $-CH_2CH_2O-R_8$ group wherein $R_8$ represents more especially a hydrogen atom, their enantiomers, diastereoisomers, N-oxides, and addition salts thereof with a pharmaceutically acceptable acid or base.

**7.** Compounds of formula (I) according to any one of claims 1 to 6, **characterised in that** n represents an integer 2, their enantiomers, diastereoisomers, N-oxides, and addition salts thereof with a pharmaceutically acceptable acid or base.

**8.** Compounds of formula (I) according to claim 1 which are :

- 2-[2-(dimethylamino)ethyl]-5-hydroxybenzo[a]pyrrolo[3,4-c]phenoxazine-1,3-dione,
- 2-[2-(diethylamino)ethyl]-5-hydroxybenzo[a]pyrrolo[3,4-c]phenoxazine-1,3-dione,
- 2-[2-(dimethylamino)ethyl]-5-(2-hydroxyethoxy)-2,3-dihydrobenzo[a]pyrrolo[3,4-c]phenoxazine-8-carbaldehyde-1,3-dione,
- 2-[2-(dimethylamino)ethyl]-5-(2-hydroxyethoxy)benzo[a]pyrrolo[3,4-c]-phenoxazine-1,3-dione,
- 2-[2-(dimethylamino)ethyl]-5-{2-[(methylsulphonyl)oxy]ethoxy}-1,2,3,8-tetrahydrobenzo[a]pyrrolo[3,4-c]phenoxazine-1,3-dione,

• 2-[2-(dimethylamino)ethyl]-5-(2-hydroxyethoxy)benzo[e]pyrido[2',3':5,6][1,4]-oxazino[3,2-g]isoindole-1,3-dione,

their enantiomers, diastereoisomers, N-oxides, and addition salts thereof with a pharmaceutically acceptable acid or base.

**9.** Process for the preparation of compounds of formula (I) according to claim 1, **characterised in that** there is used as starting material a compound of formula (II):

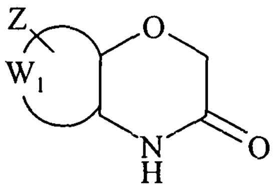

(II)

wherein $W_1$ and Z are as defined for formula (I), the amine function of which compound of formula (II) is protected by a protecting group $P_G$ well known to the person skilled in the art, to yield a compound of formula (III) :

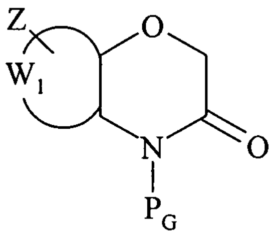

(III)

wherein $P_G$ represents a tert-butoxycarbonyl or phenoxycarbonyl group and $W_1$ and Z are as defined hereinbefore, which compound of formula (III) is treated with lithium diisopropylamide followed by diphenyl chlorophosphate to yield a compound of formula (IV) :

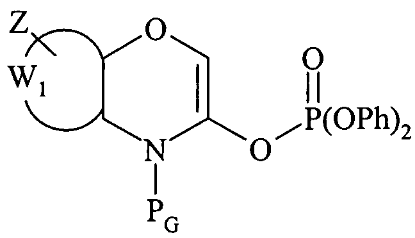

(IV)

wherein $P_G$, $W_1$ and Z are as defined hereinbefore,
which compound of formula (IV) is treated, in the presence of bis(triphenylphosphine)palladium chloride, with a compound of formula (V) :

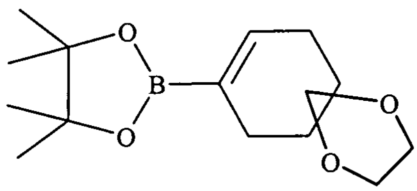

(V)

to yield a compound of formula (VI) :

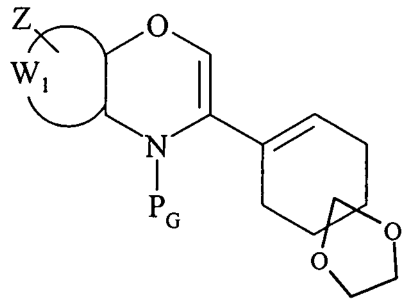

(VI)

wherein $P_G$, $W_1$ and Z are as defined hereinbefore,
which compound of formula (VI) is :

• either treated under an inert atmosphere with dimethyl acetylenedicarboxylate to yield a compound of formula (VII) :

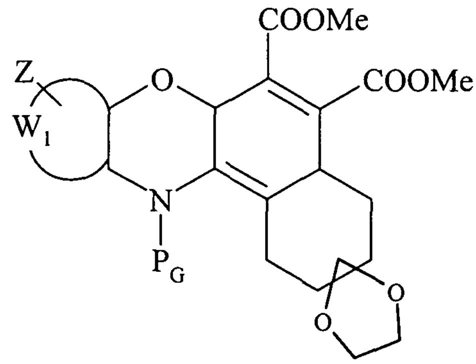

(VII)

wherein $P_G$, $W_1$ and Z are as defined hereinbefore, which compound of formula (VII) is :

♦ either treated with N-bromosuccinimide and benzoyl peroxide to yield a compound of formula (VIII) :

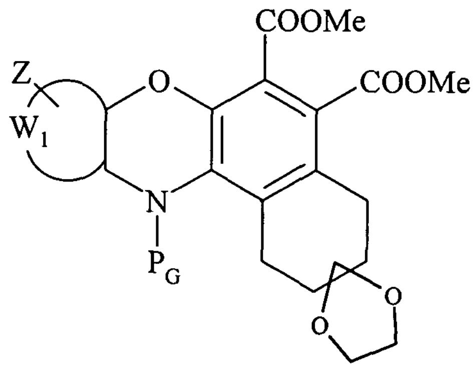

(VIII)

wherein $P_G$, $W_1$ and Z are as defined hereinbefore,
which compound of formula (VIII) is subjected to the action of hydrochloric acid to yield a compound of formula (IX):

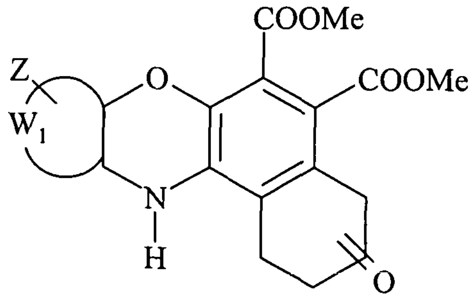

(IX)

wherein $W_1$ and Z are as defined hereinbefore, which compound of formula (IX) is subjected to the action of di-tert-butyl dicarbonate in the presence of 4-dimethylaminopyridine to yield a compound of formula (X):

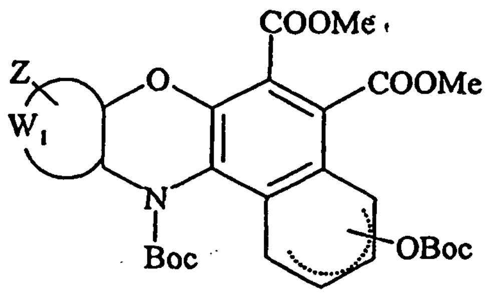

(X)

wherein ┈┈┈ represents a single or double bond, Boc represents a tert-butoxycarbonyl group and $W_1$ and Z are as defined hereinbefore,

which compound of formula (X) is subjected to the action of 2,3-dichloro-5,6-dicyano-1,4-benzoquinone to yield a compound of formula (XI) :

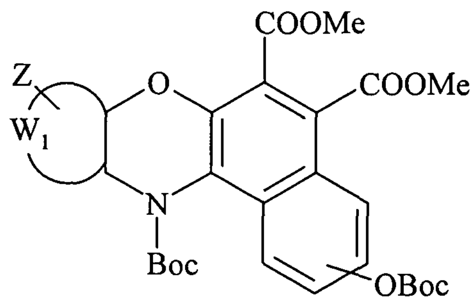

(XI)

wherein Boc, $W_1$ and Z are as defined hereinbefore,

which compound of formula (XI) is subjected to the action of sodium methanolate and is then hydrolysed to yield a compound of formula (XII) :

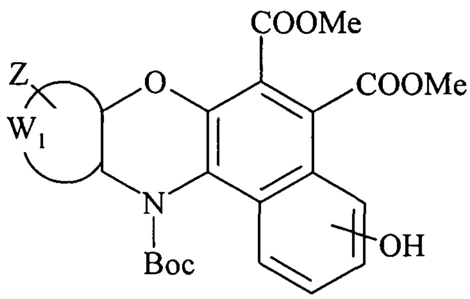

(XII)

wherein Boc, $W_1$ and Z are as defined hereinbefore, which compound of formula (XII) is subjected to the action of a compound of formula (XIII) :

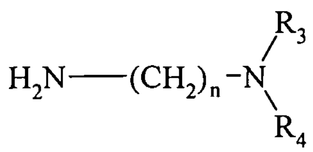

(XIII)

wherein $R_3$, $R_4$ and n are as defined for formula (I), to yield a compound of formula (I/a), a particular case of the compounds of formula (I) :

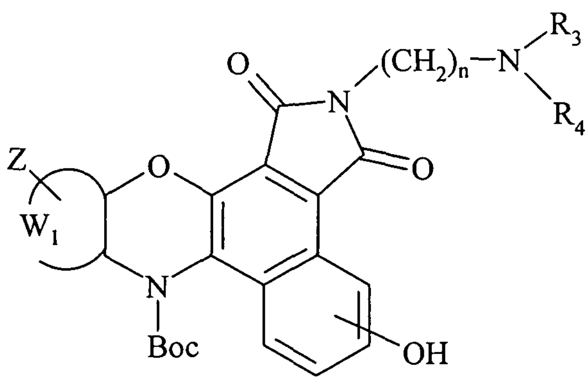

(I/a)

wherein Boc, $R_3$, $R_4$, $W_1$, Z and n are as defined hereinbefore, which compound of formula (I/a) is optionally subjected to the same reaction conditions as the compound of formula (VIII) to yield a compound of formula (I/b), a particular case of the compounds of formula (I) :

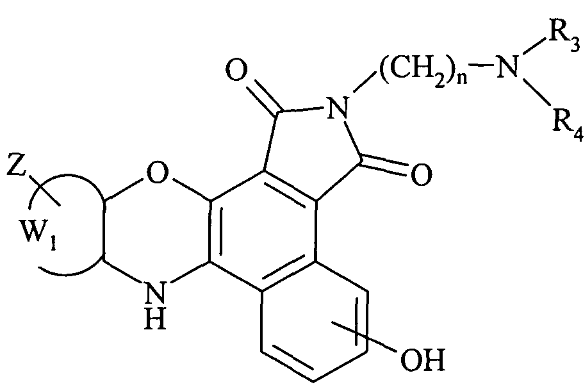

(I/b)

wherein $R_3$, $R_4$, $W_1$, Z and n are as defined hereinbefore,

♦ or subjected to the same reaction conditions as the compound of formula (X) to yield a compound of formula (XIV):

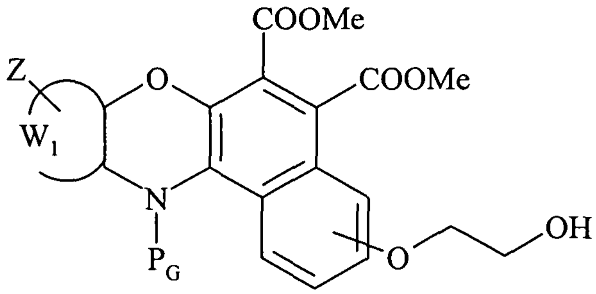

(XIV)

wherein $P_G$, $W_1$ and Z are as defined hereinbefore,

which compound of formula (XIV) is subjected to the same reaction conditions as the compound of formula (XII) to yield a compound of formula (I/c), a particular case of the compounds of formula (I) :

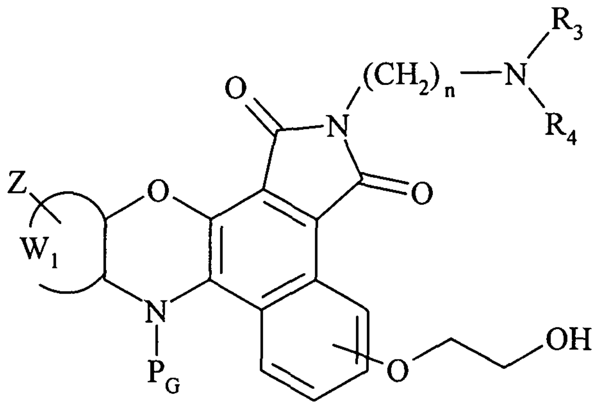

(I/c)

wherein $P_G$, $R_3$, $R_4$, $W_1$, Z and n are as defined hereinbefore, which compound of formula (I/c) is :

■ either optionally subjected to the action of formic acid to yield compounds of formulae (I/d) and (I/e), particular cases of the compounds of formula (I) :

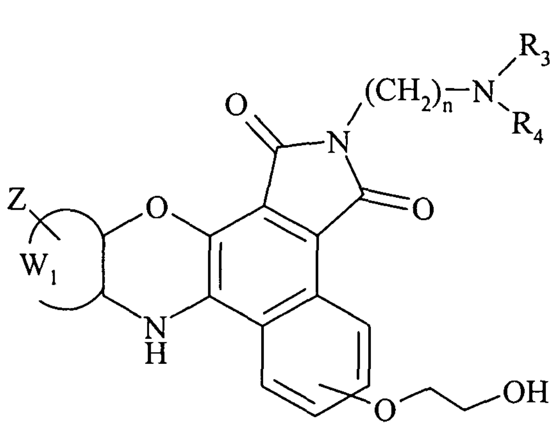

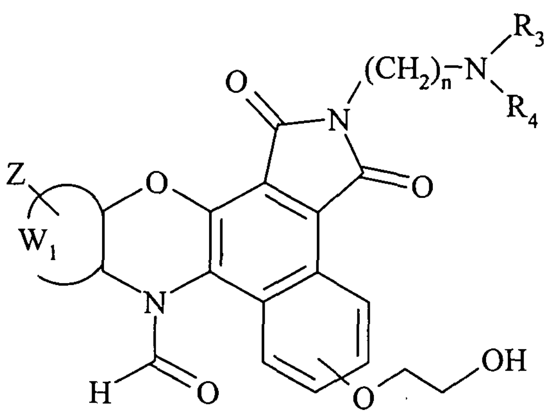

(I/d)

(I/e)

wherein $R_3$, $R_4$, $W_1$, Z and n are as defined hereinbefore,

■ or optionally subjected to the action of a compound of formula (XV) :

$R_{8a}$-G (XV)

wherein G represents a leaving group and $R_{8a}$, which is other than a hydrogen atom, has the same definition as $R_8$ in formula (I), to yield a compound of formula (I/f), a particular case of the compounds of formula (I):

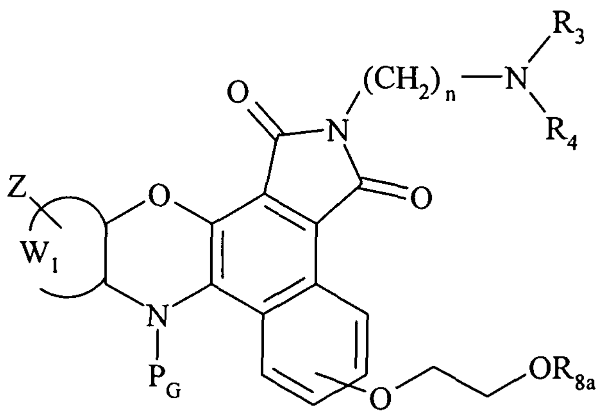

(I/f)

wherein $P_G$, $R_3$, $R_4$, $R_{8a}$, $W_1$, Z and n are as defined hereinbefore,

the amine function of which compounds of formula (I/f) is optionally deprotected according to conventional methods of organic synthesis to yield a compound of formula (I/g), a particular case of the compounds of formula (I) :

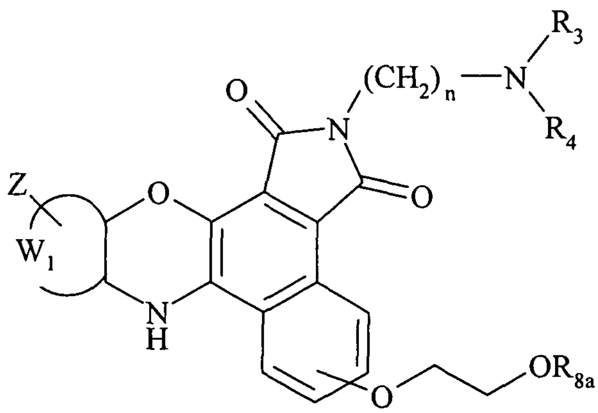

(I/g)

wherein $R_3$, $R_4$, $R_{8a}$, $W_1$, Z and n are as defined hereinbefore,

the compounds of formulae (I/b), (I/d) and (I/g) constituting the compounds of formula (I/h) :

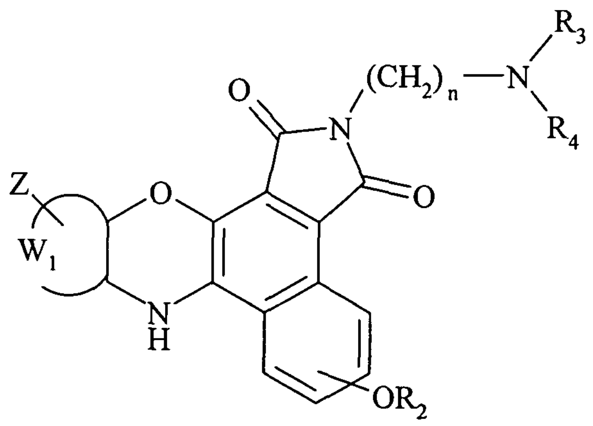

(I/h)

wherein $R_2$, $R_3$, $R_4$, $W_1$, Z and n are as defined hereinbefore,
which compounds of formula (I/h) are optionally subjected to the action of a compound of formula (XVI) :

$R_{1a}$-G (XVI)

wherein $R_{1a}$, which is other than a hydrogen atom, has the same definition as $R_1$ in formula (I) and G is as defined hereinbefore, to yield a compound of formula (I/i), a particular case of the compounds of formula (1) :

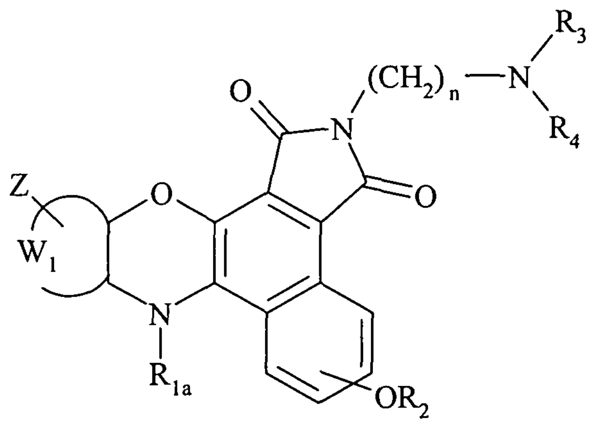

(I/i)

wherein $R_{1a}$, $R_2$, $R_3$, $R_4$, $W_1$, Z and n are as defined hereinbefore,

• or treated with N-methylmaleimide to yield a compound of formula (XVII) :

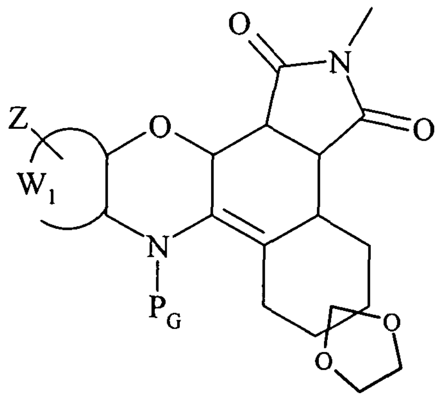

(XVII)

wherein $P_G$, $W_1$ and Z are as defined hereinbefore,
which compound of formula (XVII) is subjected to the same reaction conditions as the compound of formula (VII) to yield a compound of formula (XVIII):

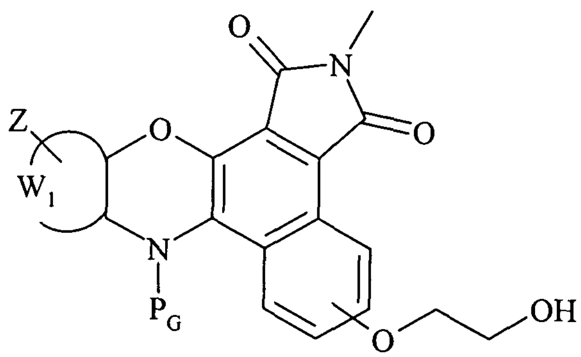

(XVIII)

wherein $P_G$, $W_1$ and Z are as defined hereinbefore,
which compound of formula (XVIII) is subjected to the same reaction conditions as the compound of formula (XII) to yield a compound of formula (I/d) as described hereinbefore,
the compounds of formulae (I/a) to (I/i) constituting the totality of the compounds of formula (I), which compounds are optionally purified according to conventional purification techniques, may, if desired, be separated into their different isomers according to a conventional separation technique and are, if desired, converted into their N-oxides and, optionally, into addition salts with a pharmaceutically acceptable acid or base.

**10.** Pharmaceutical compositions comprising as active ingredient at least one compound of formula (I) according to any one of claims 1 to 8, on its own or in combination with one or more pharmaceutically acceptable inert, non-toxic excipients or carriers.

**11.** Pharmaceutical compositions according to claim 10 for use as medicaments in the treatment of cancers.

**12.** Compounds of formula (X), (XI) and (XIV) :

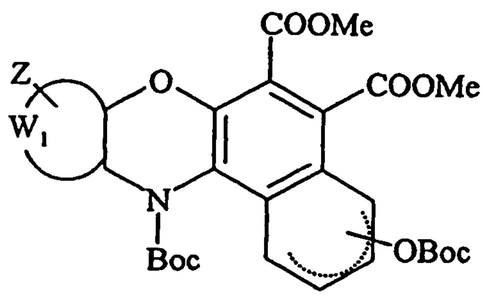

(X)

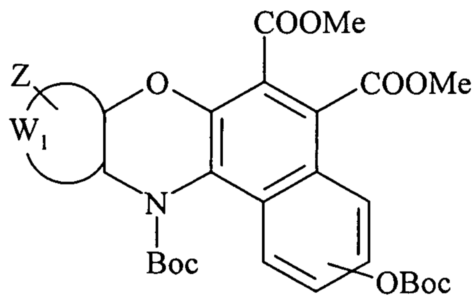

(XI)

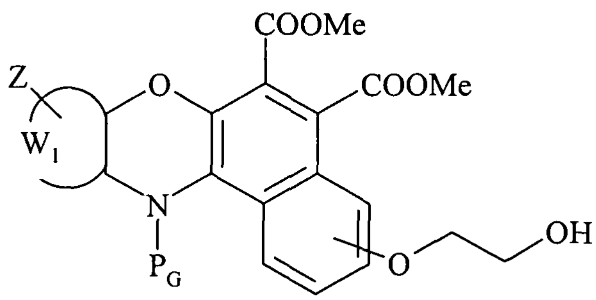

(XIV)

for use as synthesis intermediates of compounds of formula (I).

**Patentansprüche**

1. Verbindungen der Formel (I):

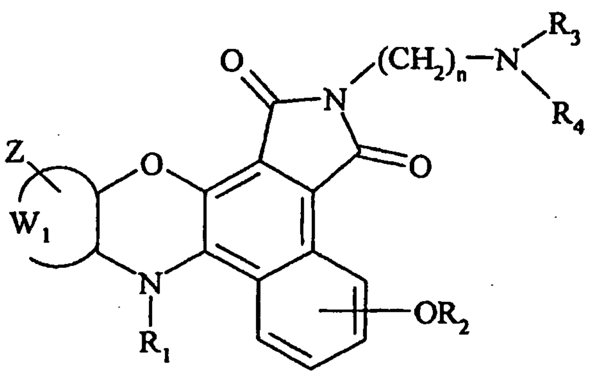

(I)

in der:

- **$W_1$** zusammen mit den Kohlenstoffatomen, an die sie gebunden ist, eine Phenylgruppe oder eine Pyridinylgruppe bedeutet,
- **Z** ein Wasserstoffatom bedeutet,
- **$R_1$** eine Gruppe bedeutet ausgewählt aus Wasserstoff und Gruppen -C(O)-$R_5$, worin:

  ⇒ $R_5$ eine Gruppe ausgewählt aus Wasserstoff, geradkettigem oder verzweigtem ($C_1$-$C_6$)-Alkoxy oder Aryloxy darstellt,

- **$R_2$** ein Wasserstoffatom oder eine Gruppe der Formel -$CH_2CH_2O$-$R_8$ bedeutet, worin:

  $R_8$ eine Gruppe darstellt ausgewählt aus Wasserstoff und Gruppen -$S(O)_t$-$R_6$ (worin $R_6$ eine geradkettige oder verzweigte ($C_1$-$C_6$)-Alkylgruppe und t 2 bedeuten),

- **$R_3$ und $R_4$,** die gleichartig oder verschieden sind, unabhängig voneinander eine geradkettige oder verzweigte ($C_1$-$C_6$)-Alkylgruppe bedeuten,
- **n** eine ganze Zahl mit einem Wert zwischen 1 und 6 einschließlich bedeutet, deren Enantiomere, Diastereoisomere, N-Oxide sowie deren Additionssalze mit einer pharmazeutisch annehmbaren Säure oder Base,

mit der Maßgabe, daß man unter Aryl eine Phenyl-, Naphthyl-, Dihydronaphthyl-, Tetrahydronaphthyl-, Indenyl- oder Indanyl-gruppe versteht, wobei jede dieser Gruppen gegebenenfalls durch eine oder mehrere, gleichartige oder verschiedenartige Gruppen substituiert ist, ausgewählt aus Halogen, geradkettigem oder verzweigtem ($C_1$-$C_6$)-Alkyl, geradkettigem oder verzweigtem ($C_1$-$C_6$)-Trihalogenalkyl, Hydroxy, geradkettigem oder verzweigtem ($C_1$-$C_6$)-Alkoxy und Amino, welches gegebenenfalls durch eine oder zwei geradkettige oder verzweigte ($C_1$-$C_6$)-Alkylgruppen substituiert ist.

2. Verbindungen der Formel (I) nach Anspruch 1, **dadurch gekennzeichnet, daß** sie Verbindungen der Formel (IA) sind:

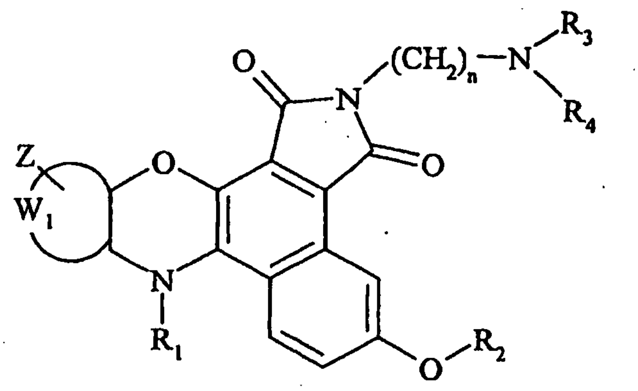

(IA)

in der $R_1$, $R_2$, $R_3$, $R_4$, $W_1$, Z und n die bezüglich der Formel (I) angegebenen Bedeutungen besitzen, deren Enantiomere, Diastereoisomere, N-Oxide sowie deren Additionssalze mit einer pharmazeutisch annehmbaren Säure oder Base.

3. Verbindungen der Formel (I) nach einem der Ansprüche 1 oder 2, **dadurch gekennzeichnet, daß** sie die Verbindungen der Formel (IB) sind:

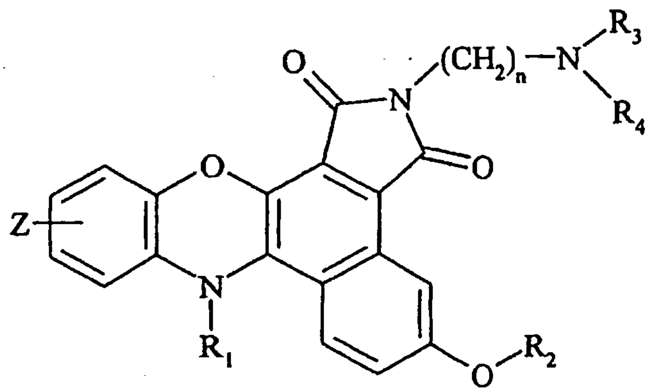

(IB)

in der $R_1$, $R_2$, $R_3$, $R_4$, $W_1$, Z und n die oben angegebenen Bedeutungen besitzen, deren Enantiomere, Diastereoisomere, N-Oxide sowie deren Additionssalze mit einer pharmazeutisch annehmbaren Säure oder Base.

**4.** Verbindungen der Formel (I) nach einem der Ansprüche 1 oder 2, **dadurch gekennzeichnet, daß** sie die Verbindungen der Formel (IC) sind:

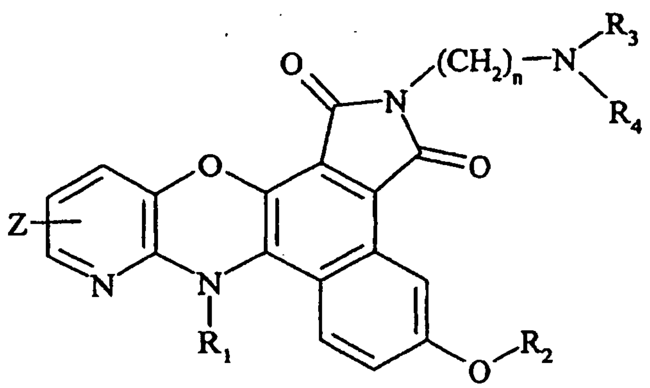

(IC)

in der $R_1$, $R_2$, $R_3$, $R_4$, $W_1$, Z und n die oben angegebenen Bedeutungen besitzen, deren Enantiomere, Diastereoisomere, N-Oxide sowie deren Additionssalze mit einer pharmazeutisch annehmbaren Säure oder Base.

**5.** Verbindungen der Formel (I) nach einem der Ansprüche 1 bis 4, **dadurch gekennzeichnet, daß** $R_1$ ein Wasserstoffatom oder eine Gruppe -C(O)-$R_5$ bedeutet, worin $R_5$ insbesondere ein Wasserstoffatom darstellt, deren Enantiomere, Diastereoisomere, N-Oxide sowie deren Additionssalze mit einer pharmazeutisch annehmbaren Säure oder Base.

**6.** Verbindungen der Formel (I) nach einem der Ansprüche 1 bis 5, **dadurch gekennzeichnet, daß** $R_2$ ein Wasserstoffatom oder eine Gruppe -$CH_2CH_2O$-$R_8$ bedeutet, worin $R_8$ insbesondere ein Wasserstoffatom darstellt, deren Enantiomere, Diastereoisomere, N-Oxide sowie deren Additionssalze mit einer pharmazeutisch annehmbaren Säure oder Base.

**7.** Verbindungen der Formel (I) nach einem der Ansprüche 1 bis 6, **dadurch gekennzeichnet, daß** n eine ganze Zahl mit einem Wert von 2 bedeutet, deren Enantiomere, Diastereoisomere, N-Oxide sowie deren Additionssalze mit einer pharmazeutisch annehmbaren Säure oder Base.

**8.** Verbindungen der Formel (I) nach Anspruch 1, nämlich:

- 2-[2-(Dimethylamino)-ethyl]-5-hydroxybenzo[a]pyrrolo[3.4-c]phenoxazin-1.3-dion,
- 2-[2-(Diethylamino)-ethyl]-5-hydroxybenzo[a]pyrrolo[3,4-c]phenoxazin-1,3-dion.
- 2-[2-(Dimethylamino)-ethyl]-5-(2-hydroxyethoxy)-2,3-dihydroxybenzo[a]pyrrolo-[3,4-c]phenoxazin-8-carbaldehyd-1,3-dion,

- 2-[2-(Dimethylamino)-ethyl]-5-(2-hydroxyethoxy)-benzo[a]pyrrolo[3.4-c]phenoxazin-1, 3-dion,
- 2-[2-(Dimethylamino)-ethyl]-5-{2-[(methylsulfonyl)-oxy]-ethoxy}-1,2,3,8-tetrahydrobenzo[a]pyrrolo[3,4-c] phenoxazin-1,3-dion und
- 2-[2-(Dimethylamino)-ethyl]-5-(2-hydroxyethoxy)-benzo[e]pyrido[2',3':5,6][1,4]-oxazino[3,2-g]isoindol-1,3-dion,

deren Enantiomere, Diastereoisomere, N-Oxide sowie deren Additionssalze mit einer pharmazeutisch annehmbaren Säure oder Base.

**9.** Verfahren zur Herstellung der Verbindungen der Formel (I) nach Anspruch 1, **dadurch gekennzeichnet, daß** man als Ausgangsprodukt eine Verbindung der Formel (II) verwendet:

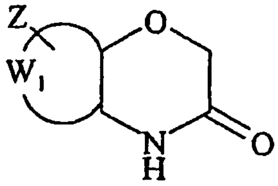

(II)

in der $W_1$ und Z die bezüglich der Formel (I) angegebenen Bedeutungen besitzen,
von welcher Verbindung der Formel (II) man die Aminfunktion mit einer dem Fachmann gut bekannten Schutzgruppe $P_G$ schützt, zur Bildung der Verbindung der Formel (III):

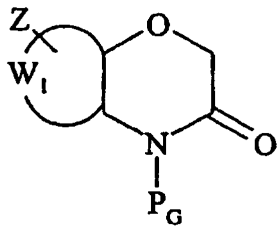

(III)

in der $P_G$ eine tert.-Butyloxycabonyl- oder Phenoxycarbonylgruppe darstellt und $W_1$ und Z die oben angegebenen Bedeutungen besitzen,
welche Verbindung der Formel (III) man mit Lithiumdiisopropylamid, gefolgt von Phenylchlorphosphat behandelt zur Bildung der Verbindung der Formel (IV):

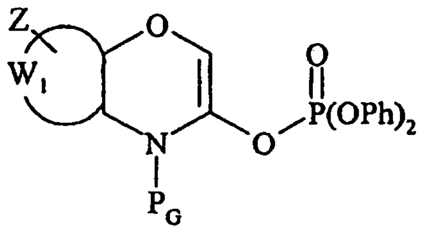

(IV)

worin $P_G$, $W_1$ und Z die oben angegebenen Bedeutungen besitzen,
welche Verbindung der Formel (IV) man in Gegenwart von Bis(triphenylphosphin)-palladium mit einer Verbindung der Formel (V) behandelt:

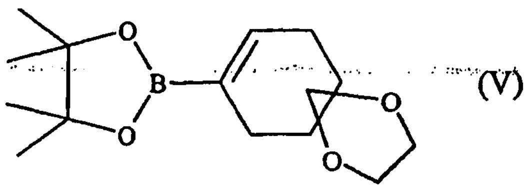

(V)

zur Bildung der Verbindung der Formel (VI):

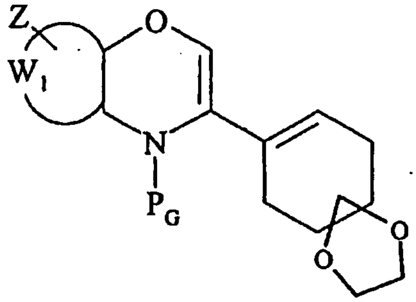

(VI)

in der $P_G$, $W_1$ und Z die oben angegebenen Bedeutungen besitzen, welche Verbndung der Formel (VI):

- entweder unter einer inerten Atmosphäre mit Acetylendicarbonsäuredimethylester behandelt wird zur Bildung der Verbindung der Formel (VII):

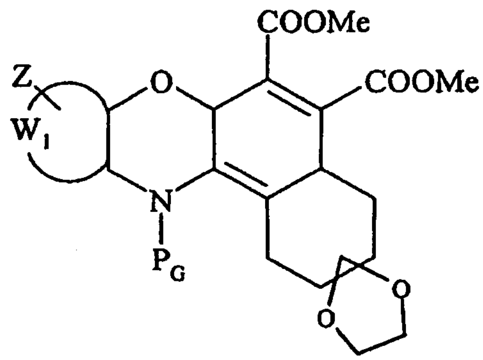

(VII)

in der $P_G$, $W_1$ und Z die oben angegebenen Bedeutungen besitzen, welche Verbindung der Formel (VII):

♦ entweder mit N-Bromsuccinimid und Benzoylperoxid behandelt wird zur Bildung der Verbindung der Formel (VIII):

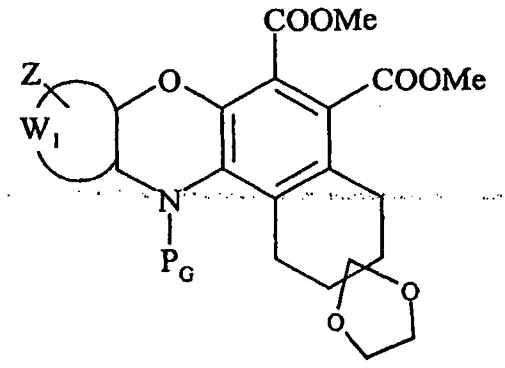

(VIII)

in der $P_G$, $W_1$ und Z die oben angegebenen Bedeutungen besitzen, welche Verbindung der Formel (VIII) der Einwirkung von Chlorwasserstoffsäure unterworfen wird zur Bildung der Verbindung der Formel (IX):

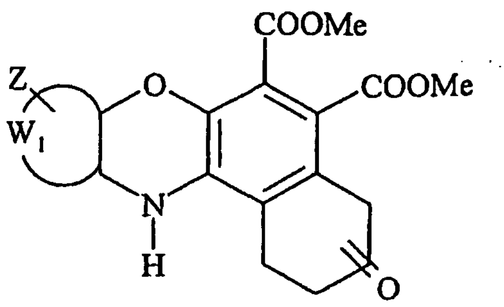

(IX)

in der $W_1$ und Z die oben angegebenen Bedeutungen besitzen, welche Verbindung der Formel (IX) in Gegenwart von 4-Dimethylaminopyridin der Einwirkung von Di-tert.-butyldicarbonat unterworfen wird zur Bildung der Verbindung der Formel (X):

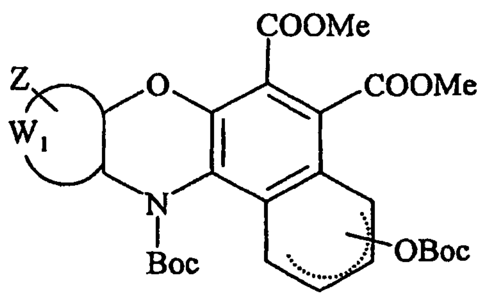

(X)

in der ..... eine Einfachbindung oder eine Doppelbindung und Boc eine tert.-Butyloxycarbonylgruppe bedeuten und $W_1$ und Z die oben angegebenen Bedeutungen besitzen, welche Verbindung der Formel (X) der Einwirkung von 2,3-Dichlor-5,6-dicyano-1,4-benzochinon unterworfen wird zur Bildung der Verbindung der Formel (XI):

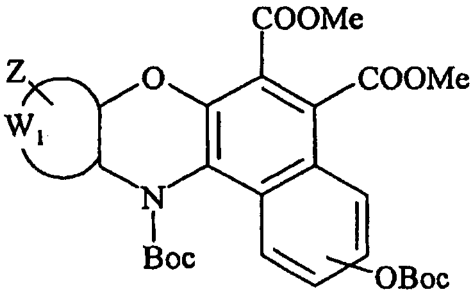

(XI)

in der Boc, $W_1$ und Z die oben angegebenen Bedeutungen besitzen,
welche Verbindung der Formel (XI) der Einwirkung von Natriummethanolat unterworfen und dann hydrolysiert wird zur Bildung der Verbindung der Formel (XII):

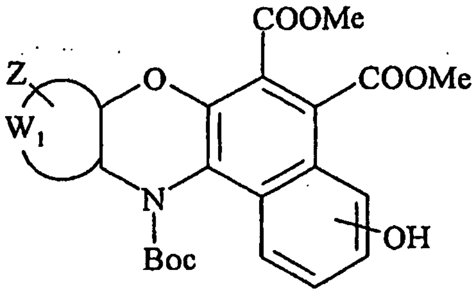

(XII)

in der Boc, $W_1$ und Z die oben angegebenen Bedeutungen besitzen,
welche Verbindung der Formel (XII) der Einwirkung einer Verbindung der Formel (XIII) unterworfen wird:

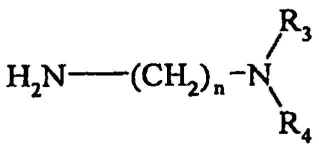

(XIII)

in der $R_3$, $R_4$ und n die bezüglich der Formel (I) angegebenen Bedeutungen besitzen,
zur Bildung der Verbindungen der Formel (I/a), einem Sonderfall der Verbindungen der Formel (1):

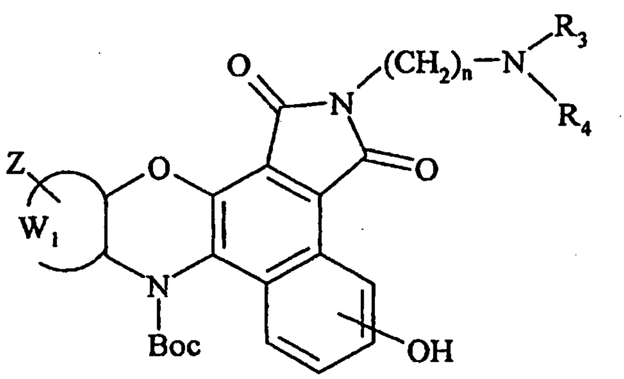

(I/a)

in der Boc, $R_3$, $R_4$, $W_1$, Z und n die oben angegebenen Bedeutungen besitzen,
welche Verbindung der Formel (I/a) gegebenenfalls den gleichen Reaktionsbedingungen unterworfen wird wie die Verbindung der Formel (VIII) zur Bildung der Verbindung der Formel (I/b), einem Sonderfall der

Verbindungen der Formel (I):

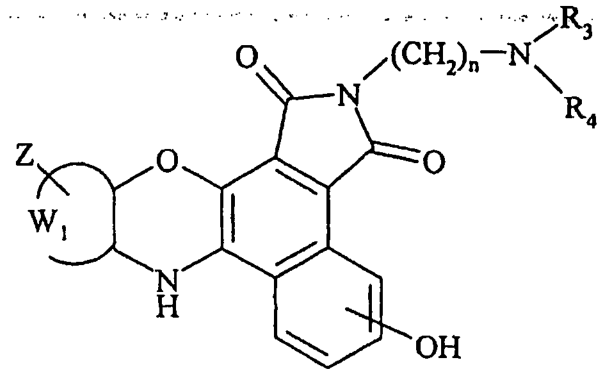

(I/b)

in der $R_3$, $R_4$, $W_1$, Z und n die oben angegebenen Bedeutungen besitzen,

♦ oder den gleichen Reaktionsbedingungen unterworfen wird wie die Verbindung der Formel (X), zur Bildung der Verbindung der Formel (XIV):

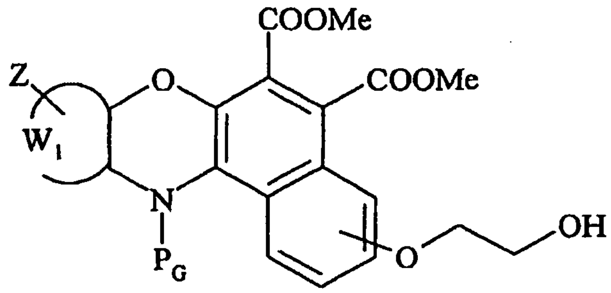

(XIV)

in der $P_G$, $W_1$ und Z die oben angegebenen Bedeutungen besitzen,
welche Verbindung der Forme (XIV) den gleichen Reaktionsbedingungen unterworfen wird wie die Verbindung der Formel (XII) zur Bildung der Verbindung der Formel (I/c), einem Sonderfall der Verbindungen der Formel (I):

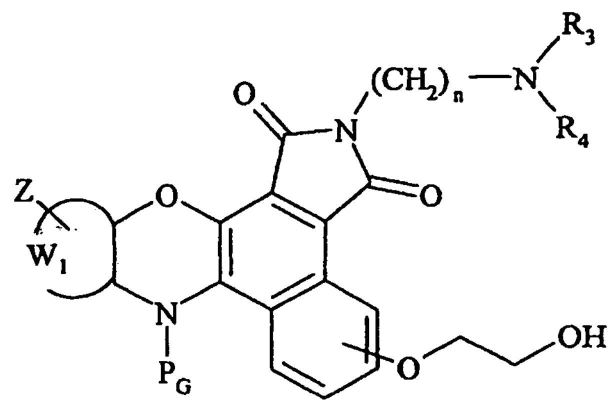

(I/c)

in der $P_G$, $R_3$, $R_4$, $W_1$, Z und n die oben angegebenen Bedeutungen besitzen,
welche Verbindung der Formel (I/c):

■ entweder gegebenenfalls der Einwirkung von Ameisensäure unterworfen wird zur Bildung der Verbindungen der Formeln (I/d) und (I/e), Sonderfällen der Verbindungen der Formel (I):

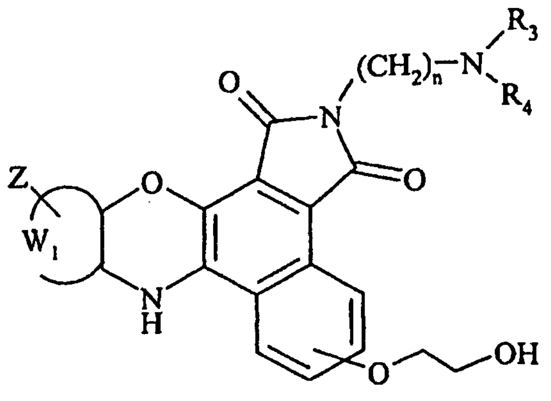

(I/d)

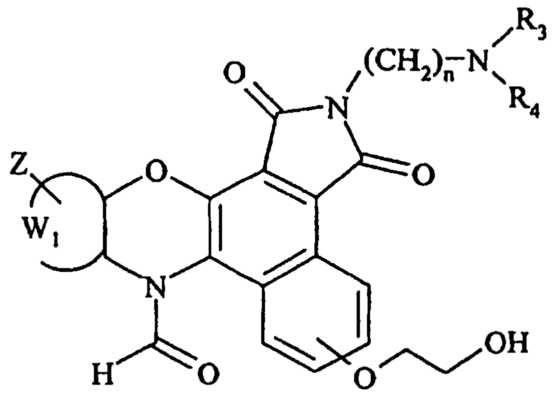

(I/e)

in den $R_3$, $R_4$, $W_1$, Z und n die oben angegebenen Bedeutungen besitzen,

■ oder gegebenenfalls der Einwirkung einer Verbindung der Formel (XV) unterworfen wird:

$R_{8a}$ - G (XV)

in der G eine austretende Gruppe darstellt und $R_{8a}$, welches von dem Wasserstoffatom verschieden ist, die gleichen Bedeutungen besitzt wie $R_1$ bezüglich der Formel (I), zur Bildung der Verbindung der Formel (I/f), einem Sonderfall der Verbindungen der Formel (I):

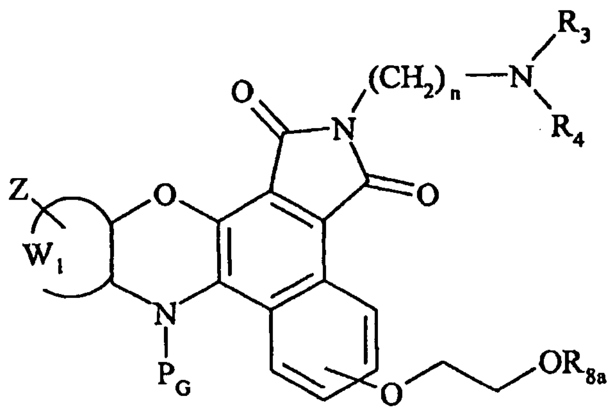

(I/f)

in der $P_G$, $R_3$, $R_4$, $R_{8a}$, $W_1$, Z und n die oben angegebenen Bedeutungen besitzen, von welchen Verbindungen der Formel (I/f) man gegebenenfalls die Schutzgruppe der Aminfunktion unter Anwendung klassischer Methoden der organischen Synthese abspaltet zur Bildung der Verbindung der Formel (I/g), einem Sonderfall der Verbindungen der Formel (I):

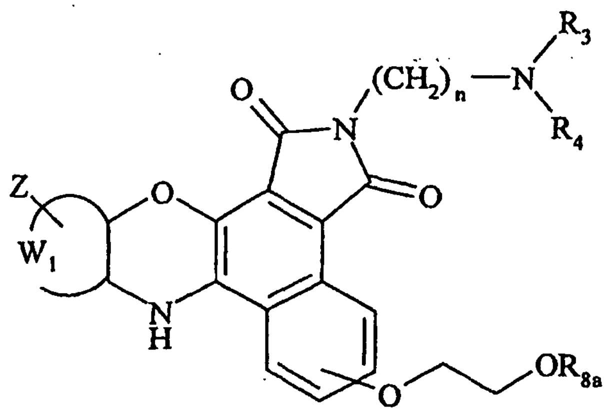

(I/g)

in der $R_3$, $R_4$, $R_{8a}$, $W_1$, Z und n die oben angegebenen Bedeutungen besitzen, welche Verbindungen der Formeln (I/b), (I/d) und (I/g) die Verbindungen der Formel (I/h) bilden:

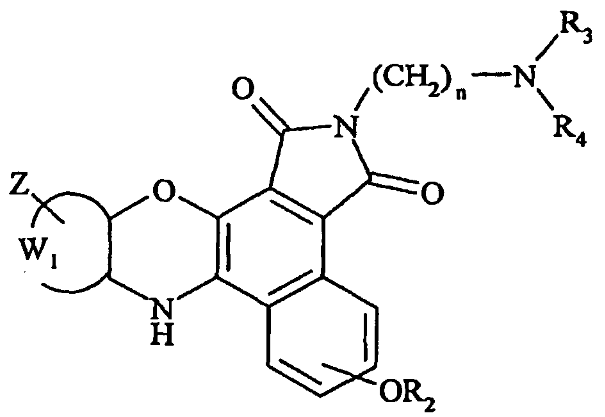

(I/h)

in der $R_2$, $R_3$, $R_4$, $W_1$, Z und n die oben angegebenen Bedeutungen besitzen, welche Verbindungen der Formel (I/h) gegebenenfalls der Einwirkung einer Verbindung der Formel (XVI) unterworfen werden:

$R_{1a}$-G (XVI)

in der $R_{1a}$, das von dem Wasserstoffatom verschieden ist, die gleichen Bedeutungen besitzt wie $R_1$ bezüglich der Formel (I) und G die oben angegebenen Bedeutungen besitzt, zur Bildung der Verbindung der Formel (I/i), einem Sonderfall der Verbindungen der Formel (I):

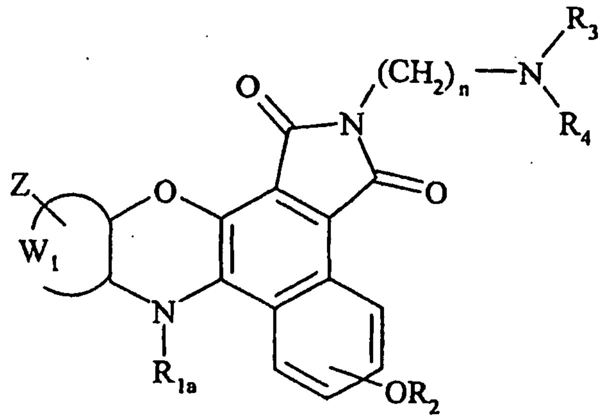

(I/i)

in der $R_{1a}$, $R_2$, $R_3$, $R_4$, $W_1$, Z und n die oben angegebenen Bedeutungen besitzen,

• oder mit N-Methylmaleinimid behandelt wird zur Bildung der Verbindung der Formel (XVII):

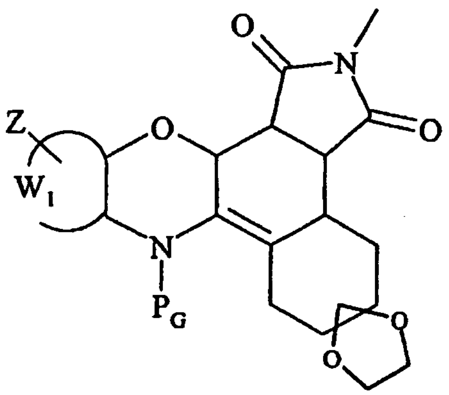

(XVII)

in der $P_G$, $W_1$ und Z die oben angegebenen Bedeutungen besitzen, welche Verbindung der Formel (XVII) den gleichen Reaktionsbedingungen unterworfen wird wie die Verbindung der Formel (VII) zur Bildung der Verbindung der Formel (XVIII):

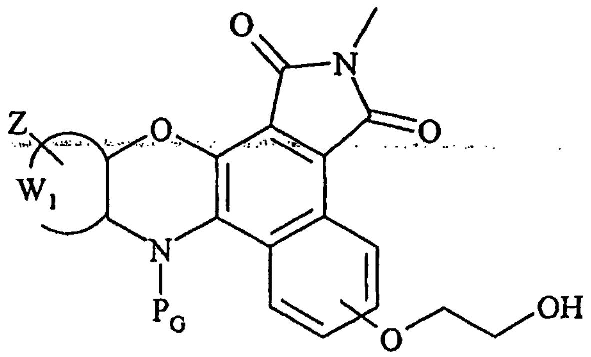

(XVIII)

in der $P_G$, $W_1$ und Z die oben angegebenen Bedeutungen besitzen, welche Verbindung der Formel (XVIII) den gleichen Reaktionsbedingungen unterworfen wird wie die Verbindung der Formel (XII) zur Bildung der Verbindung der Formel (I/d), wie sie oben definiert worden ist, welche Verbindungen der Formeln (I/a) bis (I/i), die die Gesamtheit der Verbindungen der Formel (I) bilden, man gegebenenfalls mit Hilfe klassischer Reinigungsmethoden reinigt, gewünschtenfalls mit Hilfe klassischer

Trennmethoden in ihre verschiedenen Isomeren auftrennt und gewünschtenfalls in ihre N-Oxide überführt und gegebenenfalls in ihre Säureadditionssalze mit einer pharmazeutisch annehmbaren Säure oder Base.

**10.** Pharmazeutische Zubereitungen, enthaltend als Wirkstoff mindestens eine Verbindung der Formel (I) nach einem der Ansprüche 1 bis 8, allein oder in Kombination mit einem oder mehreren inerten, nichttoxischen, pharmazeutisch annehmbaren Trägermaterialien oder Hilfsstoffen.

**11.** Pharmazeutische Zubereitungen nach Anspruch 10, nützlich als Arzneimittel bei der Behandlung von Krebs.

**12.** Verbindungen der Formeln (X), (XI) und (XIV):

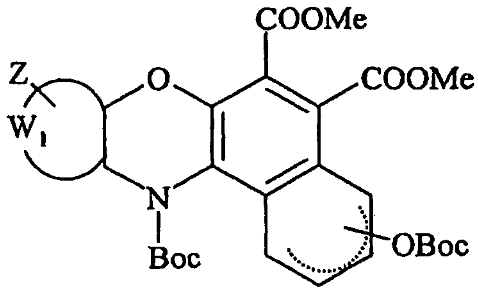

(X)

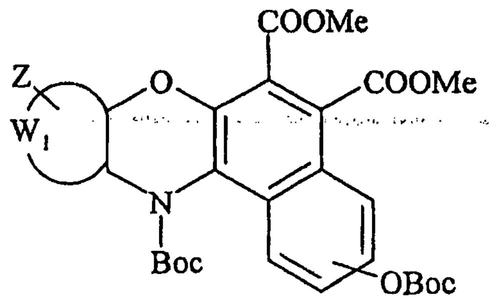

(XI)

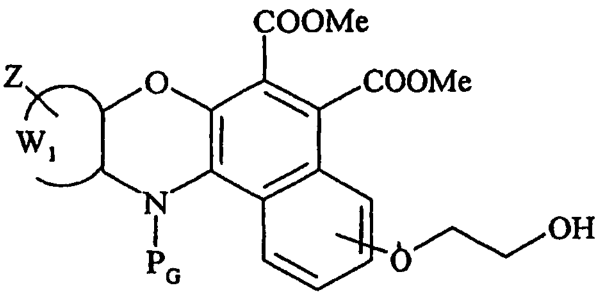

(XIV)

nützlich als Zwischenprodukte für die Synthese der Verbindungen der Formel (I).